# EUROPEAN PATENT APPLICATION

(11) **EP 4 831 298 A1**
(43) Date of publication of application: **28.10.2026**
(21) Application number: 24907215.8
(22) Date of filing: 09.12.2024
(51) Int. Cl.: C12Q 1/6827, C12N 15/09

(54) **GENETIC MUTATION EVALUATION KIT**

(30) Priority: 22.12.2023 JP 2023216494
(71) Applicant: Toyo Kohan Co., Ltd., Tokyo 141-8260 (JP); Yamaguchi University, Yamaguchi 753-8511 (JP)
(72) Inventor: MURAMATSU, Marie, Tokyo 141-8260 (JP); MORIHIRO, Junichi, Tokyo 141-8260 (JP); YUJIRI, Toshiaki, Ube-shi, Yamaguchi 755-8505 (JP)
(74) Representative: Gleiss Große Schrell und Partner mbB
(86) International application number: PCT/JP2024/043342
(87) International publication number: WO 2025/134830

(57) **Abstract**

A gene mutation in MYD88 and/or a gene mutation in CD79B are simultaneously identified. The present invention includes: a primer set for a MYD88 gene mutation and a primer set for a CD79B gene mutation; a MYD88 gene mutant-type probe and a MYD88 gene wild-type probe; and a CD79B gene mutant-type probe and a CD79B gene wild-type probe.

## Description

### Technical Field

The present invention relates to a gene mutation evaluation kit comprising: a primer set for amplifying a nucleic acid region comprising a predetermined gene mutation; and a probe for detecting an amplified nucleic acid fragment.

### Background Art

Diffuse large B-cell lymphoma (DLBCL) is the most frequent lymphoma disease type, and can occur in any organ where mature B cells physiologically exist. DLBCL has been found to be a collection of heterogeneous disease groups having diverse molecular genetic backgrounds. DLBCL is diagnosed based on an image in which large B-cell-derived tumor cells diffusely proliferate in a biopsy tissue.

Additionally, a genetic classification based on a gene mutation in DLBCL has recently been clarified. Specifically, DLBCL is classified into four subtypes: MCD subtype (L265P mutation in MYD88 and mutation in CD79B); BN2 subtype (BCL6 fusion and NOTCH2 mutation); N1 subtype (NOTCH1 mutation); and EZB subtype (EZH2 mutation and BCL2 translocation). For MCD-subtype DLBCL among these subtypes, it has been suggested that R-CHOP therapy (rituximab, cyclophosphamide, doxorubicin, vincristine, and prednisone) combined with ibrutinib, which is a kind of BTK inhibitor, is effective, as described in Non-Patent Literature 1 and Patent Literature 1.

Furthermore, lymphoplasmacytic lymphoma (LPL) is defined as a lymphoid tumor in which small B-cell lymphocytes, lymphocytes having a tendency to differentiate into plasma cells, and plasma cells are mixed, according to the WHO classification (2017), regardless of the presence or absence of an IgM-type M protein. Waldenstrom's macroglobulinemia (WM) is defined as a subset of LPL accompanied by bone marrow infiltration and IgM-type M proteinemia. In about 90% of WM/LPL patients, a mutation in the MYD88 gene is observed. Identifying the MYD88 gene mutation is useful in making a definitive diagnosis.

Incidentally, the gene mutation as described above means, in a broad sense, a state in which a gene has some abnormality, whether congenital or acquired. The gene mutation is, for example, a state in which a base constituting DNA is substituted, deleted, or added. Here, a gene is composed of a coding region that encodes a protein and a non-coding region such as an expression control region. The gene mutation exists in both the coding region and the non-coding region. Among these, the gene mutation existing in the coding region encompasses: a missense mutation in which an encoded amino acid changes; a nonsense mutation in which a codon encoding a predetermined amino acid becomes a stop codon; and a silent mutation in which the encoded amino acid does not change.

The gene mutation causes a hereditary disease or cancer, and is also associated with the medicinal efficacy of a specific drug. In addition, the gene mutation is also associated with a constitution such as obesity. Accordingly, specifying a specific gene mutation (also referred to as genotyping) has become an indispensable technique for diagnosing a hereditary disease or knowing the medicinal efficacy of a drug.

Examples of a method of genotyping include, in addition to DNA sequencing, an SSCP (Single Strand Conformation Polymorphism) method, an RFLP (Restriction Fragment Length Polymorphism) method, a PCR (Polymerase Chain Reaction) method, an AFLP (Amplified Fragment Length Polymorphism) method, an ASO (Allele Specific Oligonucleotide) probe method, a method of detecting binding to a DNA microarray or DNA beads, and the like. Among these techniques, the DNA microarray in the method using a DNA microarray usually comprises a mutant-type probe and a wild-type probe related to a gene mutation to be detected, both immobilized on a carrier.

In the method using a DNA microarray, first, a region containing the gene mutation to be detected is amplified by a nucleic acid amplification reaction using a primer having a fluorescent label. Then, a hybridization reaction is performed between the nucleic acid fragment having a fluorescent label and both the mutant-type probe and the wild-type probe. If a mutant type is comprised in the amplified nucleic acid fragment, fluorescence is observed from the mutant-type probe. Accordingly, genotyping of the gene mutation can be performed by observing the fluorescence from the mutant-type probe and the wild-type probe.

### Citation List

### Patent Literature

Patent Literature 1: JP2017-523188A

### Non-Patent Literature

Non-Patent Literature 1: Cancer Cell 39, 1643-1653, December 13, 2021

### Summary of Invention

### Technical Problem

Incidentally, a technique for simultaneously identifying the above-described gene mutation in MYD88 and/or gene mutation in CD79B is not known. If this technique becomes possible, for example, the MCD-subtype DLBCL can be easily identified. In view of this, an object of the present invention is to provide a gene mutation evaluation kit capable of simultaneously identifying a gene mutation in MYD88 and/or a gene mutation in CD79B.

### Solution to Problem

A gene mutation detecting method related to the present invention that has achieved the above-described objects encompasses the following.
(1) A gene mutation evaluation kit for use in diagnosing a disease associated with a gene mutation in the MYD88 gene and/or a gene mutation in the CD79B gene, comprising: a primer set for a MYD88 gene mutation for amplifying a nucleic acid region comprising a gene mutation in the MYD88 gene; and a primer set for a CD79B gene mutation for amplifying a nucleic acid region comprising a gene mutation in the CD79B gene; and a MYD88 gene mutant-type probe and a MYD88 gene wild-type probe related to the gene mutation in the MYD88 gene, and a CD79B gene mutant-type probe and a CD79B gene wild-type probe related to the gene mutation in the CD79B gene.
(2) The gene mutation evaluation kit of (1), wherein the gene mutation in the MYD88 gene is a substitution mutation for leucine at position 265 with proline, and the gene mutation in the CD79B gene is a missense mutation and/or nonsense mutation for the tyrosine residue at position 196 and a silent mutation for the tyrosine residue.
(3) The gene mutation evaluation kit of (1), wherein the primer set for a MYD88 gene mutation is a set of a forward primer for the MYD88 gene consisting of the base sequence of SEQ ID NO: 79 and a reverse primer for the MYD88 gene consisting of the base sequence of SEQ ID NO: 78, and the primer set for a CD79B gene mutation is a set of a forward primer for the CD79B gene consisting of the base sequence of SEQ ID NO: 85 and a reverse primer for the CD79B gene consisting of the base sequence of SEQ ID NO: 86.
(4) The gene mutation evaluation kit of (3), wherein: the reverse primer for the MYD88 gene has a label and has a concentration 2 to 6 times higher than that of the forward primer for the MYD88 gene, and the forward primer for the CD79B gene has a label and has a concentration 5 to 10 times higher than that of the reverse primer for the CD79B gene.
(5) The gene mutation evaluation kit of (1), wherein the MYD88 gene mutant-type probe, the MYD88 gene wild-type probe, the CD79B gene mutant-type probe and the CD79B gene wild-type probe comprise the base sequences shown in Table 1.

**[Table 1]**

| Probe type | Probe base sequence | SEQ ID NO |
|---|---|---|
| MYD88 gene wild-type probe | AGCGACTGATCCCCAT | 2 |
| MYD88 gene mutant-type probe | GAAGCGACCGATCCC | 6 |
| CD79B gene wild-type probe | CTTACCTCGTAGGTGTGAT | 10 |
| CD79B gene mutant-type probe M1 | CTTACCTCGTGGGTGTGA | 14 |
| CD79B gene mutant-type probe M2 | CTTACCTCGTTGGTGTGA | 19 |
| CD79B gene mutant-type probe M3 | CTTACCTCGTCGGTGTGA | 24 |
| CD79B gene mutant-type probe M4 | TTACCTCGGAGGTGTGAT | 32 |
| CD79B gene mutant-type probe M5 | CTCGCAGGTGTGATCTT | 38 |
| CD79B gene mutant-type probe M6 | CTTACCTCGAAGGTGTGAT | 41 |
| CD79B gene mutant-type probe M7 | CTTACCTCCTAGGTGTGATCT | 50 |
| CD79B gene mutant-type probe M8 | TCTCCTTACCTCTTAGGTGTG | 57 |
| CD79B gene mutant-type probe M9 | TCTCCTTACCTCATAGGTGTG | 66 |

(6) The gene mutation evaluation kit of (1), further comprising a blocking nucleic acid for MYD88 that hybridizes to a wild-type nucleic acid fragment among the nucleic acid fragments amplified with the primer set for MYD88 gene mutation, and/or a blocking nucleic acid for CD79B that hybridizes to a wild-type nucleic acid fragment among the nucleic acid fragments amplified with the primer set for CD79B gene mutation.
(7) The gene mutation evaluation kit of (6), wherein the blocking nucleic acid for MYD88 consists of the base sequence of SEQ ID NO: 89, and the blocking nucleic acid for CD79B consists of the base sequence of SEQ ID NO: 95.
(8) The gene mutation evaluation kit of (6), wherein the blocking nucleic acid for MYD88 is prepared in a hybridization solution at a concentration in the range of 0 to 500 nM, and the blocking nucleic acid for CD79B is prepared in a hybridization solution at a concentration in the range of 125 to 1000 nM.

The present specification encompasses the disclosure of Japanese Patent Application No. 2023-216494 that serves as the basis of the priority of the present application.

### Advantageous Effects of Invention

The gene mutation evaluation kit according to the present invention makes it possible that a gene mutation in MYD88 and a gene mutation in CD79B are simultaneously identified. By using the gene mutation evaluation kit according to the present invention, for example, MCD-subtype DLBCL can be easily identified based on a gene mutation in MYD88 and/or a gene mutation in CD79B.

### Brief Description of Drawings

[Figure 1] Figure 1 shows the results of measuring fluorescence intensity values when a 100% mutation model specimen was used with various probes designed in Example.
[Figure 2] Figure 2 shows the results of measuring fluorescence intensity values when a 5% mutation model specimen was used with various probes designed in Example.
[Figure 3] Figure 3 shows values for determination calculated from the fluorescence intensity values when the 5% mutation model specimen was used with various probes designed in Example.
[Figure 4] Figure 4 shows the results of measuring fluorescence intensity values and the results of calculating values for determination when a wild-type sample, a 5% mutation model specimen, or genomic DNA was used with a wild-type probe (gene mutation in the MYD88 gene) designed in Example.
[Figure 5] Figure 5 shows the results of measuring fluorescence intensity values when a wild-type sample, a 5% mutation model specimen, or genomic DNA was used with a wild-type probe (gene mutation in the CD79B gene) designed in Example.
[Figure 6] Figure 6 shows values for determination calculated from the fluorescence intensity values when a wild-type sample, a 5% mutation model specimen, or genomic DNA was used with the wild-type probe (gene mutation in the CD79B gene) designed in Example.
[Figure 7] Figure 7 shows the results (values for determination) of detecting gene mutations in genomic DNA using various probes selected in Example.
[Figure 8] Figure 8 shows combinations tested in Example, each combination composed of a primer set for amplifying a region comprising a gene mutation in the CD79B gene and a primer set for amplifying a region comprising a gene mutation in the MYD88 gene.
[Figure 9] Figure 9 shows a relationship between a nucleic acid amplification amount and an annealing temperature for each combination of the primer sets.
[Figure 10] Figure 10 shows the results of confirming nucleic acid fragments comprised in a reaction liquid by electrophoresis with respect to combinations [5], [6], and [7] of the primer sets.
[Figure 11] Figure 11 shows the results of performing PCR with the combinations [5], [6], and [7] of the primer sets and detecting amplified fragments by fluorescence intensities from the wild-type probe and the mutant-type probe.
[Figure 12] Figure 12 shows the results of measuring fluorescence intensities from combinations [6] and [7] of the primer sets while changing a mixing ratio of a labeled primer and an unlabeled primer.
[Figure 13] Figure 13 shows a concentration ratio of a forward primer and a reverse primer comprised in the primer set tested in Example.
[Figure 14] Figure 14 shows a relationship between a concentration ratio of a forward primer and a reverse primer comprised in the primer set (MYD88 gene) tested in Example and a fluorescence intensity value.
[Figure 15] Figure 15 shows a relationship between a concentration ratio of a forward primer and a reverse primer comprised in the primer set (CD79B gene) tested in Example and a fluorescence intensity value.
[Figure 16] Figure 16 shows the results of measuring fluorescence intensity from the mutant-type probe when a blocking nucleic acid (MYD88 gene) designed in Example was used.
[Figure 17] Figure 17 shows the results of measuring fluorescence intensities from the wild-type probe and the mutant-type probe when v2-2 or v2-3 among the blocking nucleic acids (MYD88 gene) designed in Example was used.
[Figure 18] Figure 18 shows values for determination calculated from fluorescence intensities measured with the wild-type probe and the mutant-type probe when v2-2 or v2-3 among the blocking nucleic acids (MYD88 gene) designed in Example was used.
[Figure 19] Figure 19 shows values for determination calculated from fluorescence intensities measured with mutant-type probes when blocking nucleic acids (CD79B gene) designed in Example were used.
[Figure 20] Figure 20 shows values for determination calculated with the mutant-type probes, using v4-1 and v4-2 among the blocking nucleic acids (CD79B gene) designed in Example, and using a wild-type model specimen or a 5% mutation model specimen.
[Figure 21] Figure 21 shows fluorescence intensity values calculated with the mutant-type probes, using v4-1 and v4-2 among the blocking nucleic acids (CD79B gene) designed in Example, and using a wild-type model specimen or a 5% mutation model specimen.
[Figure 22] Figure 22 shows the results of calculating values for determination while changing the concentration of the blocking nucleic acid (CD79B gene) v4-2 designed in Example.
[Figure 23] Figure 23 shows the results of measuring fluorescence intensity from each mutant-type probe when the concentration of the blocking nucleic acid (CD79B gene) v4-2 designed in Example was set to 500 nM, 750 nM, or 1000 nM, and the 5% mutation model specimen was used.
[Figure 24] Figure 24 shows the results of calculating values for determination of each mutant-type probe when the concentration of the blocking nucleic acid (CD79B gene) v4-2 designed in Example was 500 nM or 750 nM, and the wild-type model specimen or the 5% mutation model specimen was used.

### Description of Embodiments

The gene mutation evaluation kit according to the present invention will be described in detail below. The gene mutation evaluation kit simultaneously identifies at least a gene mutation in the MYD88 gene and a gene mutation in the CD79B gene. Accordingly, the gene mutation evaluation kit can be used for diagnosing a disease associated with a gene mutation in the MYD88 gene and/or a gene mutation in the CD79B gene. Note that the MYD88 gene and the CD79B gene are genes encoding proteins associated with an NF-κB signal transduction pathway. A gene mutation in these genes is thought to constitutively activate the NF-κB pathway, leading to the occurrence of lymphoma and the like. For example, the MYD88 L265P mutation is frequently observed in lymphoplasmacytic lymphoma (LPL)/Waldenstrom's macroglobulinemia (WM). Additionally, the MYD88 L265P mutation and the CD79B Y196 mutation are frequently observed in diffuse large B-cell lymphoma (DLBCL).

Here, the gene mutation evaluation kit comprises: a primer set for amplifying a nucleic acid region comprising a gene mutation; and a probe set for detecting an amplified nucleic acid fragment. That is, the gene mutation evaluation kit comprises: a primer set for a MYD88 gene mutation for amplifying a nucleic acid region comprising a gene mutation in the MYD88 gene; and a primer set for a CD79B gene mutation for amplifying a nucleic acid region comprising a gene mutation in the CD79B gene. Additionally, the gene mutation evaluation kit comprises: a probe set consisting of a MYD88 gene mutant-type probe and a MYD88 gene wild-type probe related to the gene mutation in the MYD88 gene; and a probe set consisting of a CD79B gene mutant-type probe and a CD79B gene wild-type probe related to the gene mutation in the CD79B gene.

MYD88 is a cytoplasmic adapter protein that plays a central role in innate and adaptive immune responses, and functions as an essential signaling substance in interleukin-1 and Toll-like receptor signal transduction pathways. MYD88 is also called IMD68, MYD88D, and WM1. A gene mutation in the MYD88 gene is a missense mutation caused by a base substitution in the codon (CTG) encoding leucine at position 265 from the N-terminus, resulting in a change to proline. The missense mutation is caused by a mutation (794T>C) in which thymine at position 794 from the 5' end of a region encoding MYD88 is substituted with cytosine.

On the other hand, CD79B exists as a heterodimeric complex with CD79A, and associates with a cell surface immunoglobulin to form a B-cell antigen receptor. CD79B is also called B29, Ig-β, or AGM6. A gene mutation in the CD79B gene means that a base substitution occurs in the codon (TAC) encoding tyrosine located at position 196 from the N-terminus, resulting in any of three types of gene mutations: a missense mutation resulting in a different amino acid; a nonsense mutation resulting in a stop codon; and a silent mutation in which the encoded amino acid remains tyrosine.

Here, the missense mutation in the CD79B gene is a mutation in which thymine at position 589 from the 5' end of a region encoding CD79B is substituted with cytosine (the mutation is described as 589T>C, and similar mutations are hereinafter described with A: adenine, G: guanine, C: cytosine, and T: thymine), 589T>A, 589T>G, 590A>C, 590A>G, or 590A>T. In addition, the nonsense mutations in the CD79B gene are 591C>G and 591C>A. Furthermore, the silent mutation in the CD79B gene is 591C>T.

These mutations to thymine at position 196 in the CD79B gene are collectively shown in Table 2. Note that in Table 2, the wild-type sequence is designated as W in the Type column, M1 to M6 in the same column are missense mutations, M7 and M8 are nonsense mutations, and M9 is a silent mutation.

**[Table 2]**

| Type | Amino acid | Base | | |
|---|---|---|---|---|
| | | 589 | 590 | 591 |
| W | Y196 | T | A | C |
| M1 | Y196H | C | A | C |
| M2 | Y196N | A | A | C |
| M3 | Y196D | G | A | C |
| M4 | Y196S | T | C | C |
| M5 | Y196C | T | G | C |
| M6 | Y196F | T | T | C |
| M7 | Y196* (STOP) | T | A | G |
| M8 | Y196* (STOP) | T | A | A |
| M9 | Y196= | T | A | T |

### 1) Primer set

The primer set comprised in the gene mutation evaluation kit according to the present invention comprises: a primer set for a MYD88 gene mutation for amplifying a nucleic acid region comprising a gene mutation in the MYD88 gene; and a primer set for a CD79B gene mutation for amplifying a nucleic acid region comprising a gene mutation in the CD79B gene.

As shown in the below-described Example, the primer set for a MYD88 gene mutation and the primer set for a CD79B gene mutation are designed based on, for example, the conditions that a nucleic acid fragment in a desired amount is amplified by each primer set in the same reaction system, and that non-specific amplification is low. Specifically, the primer set is designed such that the amounts (molar concentrations) of nucleic acid fragments obtained are close to each other when a nucleic acid amplification reaction is performed using a plasmid comprising a nucleic acid region to be amplified, as a template and when a nucleic acid amplification reaction is performed using genomic DNA comprising a nucleic acid region to be amplified, as a template. Additionally, after performing the nucleic acid amplification reaction, the nucleic acid fragment comprised in the reaction liquid is detected by a technique such as electrophoresis, whereby a primer set having low non-specific amplification can be designed.

As an example, the primer set for a MYD88 gene mutation may be a set of a forward primer for the MYD88 gene consisting of the base sequence of SEQ ID NO: 79 (5'-CAGGTGCCCATCAGAAG-3') and a reverse primer for the MYD88 gene consisting of the base sequence of SEQ ID NO: 78 (5'-CGCAGACAGTGATGAACC-3'), and the primer set for a CD79B gene mutation may be a set of a forward primer for the CD79B gene consisting of the base sequence of SEQ ID NO: 85 (5'-CAGGATGACAGCAAGGC-3') and a reverse primer for the CD79B gene consisting of the base sequence of SEQ ID NO: 86 (5'-CAACCACACCAGCAGATAG-3').

The primer set defined by these base sequences is designed such that the Tm values of the forward primer and the reverse primer become close to each other. Accordingly, a base sequence in which one base or a plurality of bases are added to a 5' end and/or a 3' end, or a base sequence in which one base or a plurality of bases at the 5' end and/or the 3' end are deleted may be used within a range in which a difference between the Tm values of the forward primer and the reverse primer does not become large.

The Tm value of the primer can be calculated by a nearest neighbor method or software applying the nearest neighbor method. The difference between the Tm values of the forward primer and the reverse primer is set to, for example, within 3°C, preferably within 2.5°C, more preferably within 2.0°C, still more preferably within 1.5°C, still more preferably within 1.0°C, and still more preferably within 0.5°C.

The base length of the primer can usually be 17 to 25 bases. Accordingly, when one base or a plurality of bases are added to the 5' end and/or the 3' end of the above-described specific base sequence, the length is preferably within this range. Additionally, the one base or the plurality of bases to be added can be determined based on the base sequence (NCBI Gene ID: 4615) of the MYD88 gene and the base sequence (NCBI Gene ID: 974) of the CD79B gene that are registered in a known database.

Additionally, both the primer set for a MYD88 gene mutation and the primer set for a CD79B gene mutation preferably have a label on any one of the forward primer and the reverse primer. Because the forward primer or the reverse primer has a label, a nucleic acid fragment having the label can be amplified. Although not particularly limited, the reverse primer for the MYD88 gene in the primer set for a MYD88 gene mutation can have a label, and the forward primer for the CD79B gene in the primer set for a CD79B gene mutation can have a label.

Here, the label means, but is not particularly limited to, binding a labeling substance such as a radioisotope, a fluorescent dye, or an organic compound such as digoxigenin (DIG) or biotin to the above-described primer. As the labeling substance, any of various conventionally known labeling substances can be suitably selected and used. In particular, it is preferable to use a fluorescent dye such as an indocyanine compound (IC5-OSu, IC5-PE-maleimide, IC3-OSu, or IC3-PE-maleimide), BDP, a cyanine dye, a fluorescein dye, a pyrene dye, and a rhodamine dye, which can be detected easily and with high sensitivity.

Furthermore, a ratio (concentration ratio) of the forward primer to the reverse primer comprised in the primer set for a MYD88 gene mutation is not particularly limited, but it is preferable that the ratio of the primer having a label is larger than the ratio of the primer having no label. For example, when the reverse primer has a label, the ratio of the reverse primer to the forward primer (reverse primer concentration: forward primer concentration) can be 15:10, is preferably 20:10, is more preferably 30:10, and is most preferably 40:10. Note that the ratio (reverse primer concentration: forward primer concentration) may be 50:10 or 60:10. That is, when the reverse primer has a label, the concentration of the reverse primer can be 1.5 to 6 times, is particularly preferably 2 to 6 times, is more preferably 3 to 5 times, and is most preferably 4 times the concentration of the forward primer. In this case, a most excellent fluorescence intensity value was achieved. By setting such a ratio, fluorescence intensity derived from an amplification product obtained with the primer set for a MYD88 gene mutation can be increased.

Additionally, a ratio (concentration ratio) of the forward primer to the reverse primer comprised in the primer set for a CD79B gene mutation is not particularly limited, but it is preferable that the ratio of the primer having a label is larger than the ratio of the primer having no label. For example, when the forward primer has a label, the ratio of the forward primer to the reverse primer (forward primer concentration: reverse primer concentration) can be 15:10, 20:10, 30:10, 40:10, and 50:10. Additionally, the ratio is preferably 60:10, and more preferably 70:10. Furthermore, the ratio can be 80:10, 90:10, and 100:10. That is, when the forward primer has a label, the concentration of the forward primer can be 1.5 to 10 times, is particularly preferably 5 to 10 times, is more preferably 6 to 7 times, and is most preferably 7 times the concentration of the reverse primer. By setting such a ratio, fluorescence intensity derived from an amplification product obtained with the primer set for a CD79B gene mutation can be increased.

### 2) Probe

The probe set comprised in the gene mutation evaluation kit according to the present invention comprises a probe set related to a gene mutation in the MYD88 gene and a probe set related to a gene mutation in the CD79B gene. Each probe set comprises a mutant-type probe corresponding to a mutant type in the gene mutation to be detected and a wild-type probe corresponding to a wild type in the gene mutation. Note that, when the gene mutation to be detected has a plurality of patterns of mutant types, a plurality of mutant-type probes corresponding to all of the plurality of patterns of mutant types may be prepared, or one or a plurality of mutant-type probes corresponding to specific mutant types among the plurality of patterns of mutant types may be prepared.

Hereinafter, each probe set will be described, and the mutant-type probe and the wild-type probe comprised in these probe sets are preferably nucleic acids, more preferably DNA. DNA encompasses both a double strand and a single strand, but is preferably single-stranded DNA. The mutant-type probe and the wild-type probe can be obtained by, for example, chemical synthesis using a nucleic acid synthesizer. As the nucleic acid synthesizer, a device called a DNA synthesizer, a fully automatic nucleic acid synthesizer, an automatic nucleic acid synthesizer, or the like can be used.

### Probe set related to a gene mutation in the MYD88 gene

A probe set related to a gene mutation in the MYD88 gene can be designed based on the base sequence of a nucleic acid fragment amplified by the nucleic acid amplification reaction using the above-described primer set for a MYD88 gene mutation. That is, the mutant-type probe and the wild-type probe comprised in the probe set can be designed as a base sequence complementary to a region comprising a site of the gene mutation comprised in the amplified nucleic acid fragment. As described above, the wild type in the gene mutation in the MYD88 gene has T at the 794th base, and the mutant type in the gene mutation has C at the 794th base. Accordingly, the wild-type probe and the mutant-type probe comprise a base corresponding to the 794th base.

The wild-type probe and the mutant-type probe may be designed such that only the base corresponding to the gene mutation is different and the rest are all identically matched, but the probes may be designed to have different base lengths or different 3' ends and/or 5' ends. The wild-type probe and the mutant-type probe can be, but are not particularly limited to, for example, 10 to 30 bases in length, and preferably 15 to 25 bases in length. Additionally, in the wild-type probe and the mutant-type probe, the position of the base corresponding to the gene mutation is preferably at a center of a character string when the bases constituting the wild-type probe and the mutant-type probe are viewed as the character string. Note that in the case of a wild-type probe and a mutant-type probe consisting of an even number of bases, the center of the character string is meant to comprise a case of being shifted by one in the 5' end or 3' end direction.

More specifically, regarding a nucleic acid fragment obtained by a nucleic acid amplification reaction using the above-described set of the forward primer for the MYD88 gene consisting of the base sequence of SEQ ID NO: 79 (5'-CAGGTGCCCATCAGAAG-3') and the reverse primer for the MYD88 gene consisting of the base sequence of SEQ ID NO: 78 (5'-CGCAGACAGTGATGAACC-3'), a MYD88 gene wild-type probe consisting of the base sequence of SEQ ID NO: 2 (5'-AGCGACTGATCCCCAT-3') and a MYD88 gene mutant-type probe consisting of the base sequence of SEQ ID NO: 6 (5'-GAAGCGACCGATCCC-3') can be designed. According to the MYD88 gene wild-type probe and the MYD88 gene mutant-type probe, fluorescence intensity derived from the amplified nucleic acid fragment is high, the separation performance is high, and thus, the gene mutation to be tested can be detected with high precision. Here, the separation performance means the difference between the values for determination for the respective samples, which values are calculated according to the following Formula 1 after amplifying nucleic acid fragments using samples having different mutation ratios and detecting each of the nucleic acid fragments with a wild-type probe and a mutant-type probe. As samples having different mutation ratios, a 100% wild-type sample and a 5% mutant type sample can be used. Formula 1: 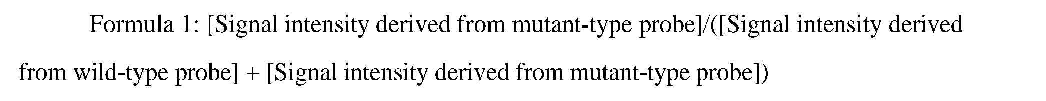 [Signal intensity derived from mutant-type probe]/([Signal intensity derived from wild-type probe] + [Signal intensity derived from mutant-type probe])

### Probe set related to a gene mutation in the CD79B gene

A probe set related to a gene mutation in the CD79B gene can be designed based on the base sequence of a nucleic acid fragment amplified by the nucleic acid amplification reaction using the above-described primer set for a CD79B gene mutation. That is, the mutant-type probe and the wild-type probe comprised in the probe set can be designed as a base sequence complementary to a region comprising a site of the gene mutation comprised in the amplified nucleic acid fragment. As described above, the wild type in the gene mutation in the CD79B gene has T at the 589th base, A at the 590th base, and C at the 591st base. Additionally, as shown in Table 2 above, the mutant types in the gene mutation comprise missense mutations denoted as M1 to M6, nonsense mutations denoted as M7 and M8, and a silent mutation denoted as M9.

The wild-type probe and the mutant-type probe may be designed such that only the base corresponding to the gene mutation is different and the rest are all identically matched, but the probes may be designed to have different base lengths or different 3' ends and/or 5' ends. The wild-type probe and the mutant-type probe can be, but are not particularly limited to, for example, 10 to 30 bases in length, and preferably 15 to 25 bases in length. Additionally, in the wild-type probe and the mutant-type probe, the position of the base corresponding to the gene mutation is preferably at a center of a character string when the bases constituting the wild-type probe and the mutant-type probe are viewed as the character string. Note that in the case of a wild-type probe and a mutant-type probe consisting of an even number of bases, the center of the character string is meant to comprise a case of being shifted by one base in the 5' end or 3' end direction.

Regarding the gene mutation in the CD79B gene, a mutant-type probe is designed for each of the above-described nine kinds of mutant types. More specifically, regarding a nucleic acid fragment obtained by a nucleic acid amplification reaction using the above-described set of the forward primer for the CD79B gene consisting of the base sequence of SEQ ID NO: 85 (5'-CAGGATGACAGCAAGGC-3') and the reverse primer for the CD79B gene consisting of the base sequence of SEQ ID NO: 86 (5'-CAACCACACCAGCAGATAG-3'), probes consisting of base sequences shown in Table 3 can be designed, although not particularly limited. Note that, in the probe base sequences described in Table 3, a portion corresponding to a codon encoding an amino acid at position 196 from the N-terminus is underlined.

**[Table 3]**

| Wild type (W), Mutant type (M) | Probe base sequence | SEQ ID NO |
|---|---|---|
| W | CTTACCTCGTAGGTGTGAT | 10 |
| M1 | CTTACCTCGTGGGTGTGA | 14 |
| M2 | CTTACCTCGTTGGTGTGA | 19 |
| M3 | CTTACCTCGTCGGTGTGA | 24 |
| M4 | TTACCTCGGAGGTGTGAT | 32 |
| M5 | CTCGCAGGTGTGATCTT | 38 |
| M6 | CTTACCTCGAAGGTGTGAT | 41 |
| M7 | CTTACCTCCTAGGTGTGATCT | 50 |
| M8 | TCTCCTTACCTCTTAGGTGTG | 57 |
| M9 | TCTCCTTACCTCATAGGTGTG | 66 |

Note that in the following description, a mutant-type probe for detecting missense mutations (M1 to M6) and nonsense mutations (M7 and M8) is referred to as a first probe in some cases, and a probe for detecting a silent mutation (M9) is referred to as a second probe in some cases.

According to the wild-type probe and the mutant-type probe designed for the gene mutation in the CD79B gene as shown in Table 3, fluorescence intensity derived from the amplified nucleic acid fragment is high, the separation performance is high, and thus, the gene mutation to be tested can be detected with high precision. Here, the separation performance (degree of separation) means the difference between the values for determination for the respective samples, which values are calculated according to the following Formula 1 after amplifying nucleic acid fragments using samples having different mutation ratios and detecting each of the nucleic acid fragments with a wild-type probe and a mutant-type probe. As samples having different mutation ratios, a 100% wild-type sample and a 5% mutant sample can be used. Formula 1: 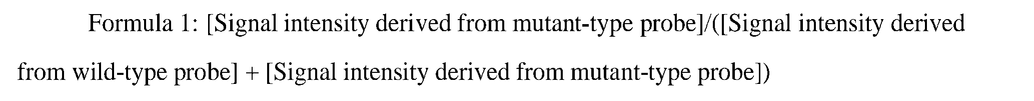 [Signal intensity derived from mutant-type probe]/([Signal intensity derived from wild-type probe] + [Signal intensity derived from mutant-type probe])

### DNA chip (DNA microarray)

The wild-type probe and the mutant-type probe designed as described above are preferably used in the form of a microarray (as an example, a DNA chip) with the 5' end thereof immobilized on a carrier. In this case, the microarray has a first probe, a second probe, and a wild-type probe for a gene mutation to be tested. The microarray may have a wild-type probe and a mutant-type probe related to a gene mutation in the MYD88 gene and a gene mutation in the CD79B gene respectively. Alternatively, a microarray having a wild-type probe and a mutant-type probe related to a gene mutation in the MYD88 gene and a microarray having a wild-type probe and a mutant-type probe related to a gene mutation in the CD79B gene may be prepared.

As a material of the carrier, a material known in the art can be used without particular limitation. Examples thereof include: noble metals such as platinum, platinum black, gold, palladium, rhodium, silver, mercury, tungsten, and a compound thereof, and conductor materials of carbon or the like, typified by graphite and a carbon fiber; silicon materials typified by single crystal silicon, amorphous silicon, silicon carbide, silicon oxide, and silicon nitride, and a composite material of these silicon materials typified by SOI (silicon on insulator) and the like; an inorganic material such as glass, quartz glass, alumina, sapphire, ceramics, forsterite, and photosensitive glass; and organic materials such as polyethylene, ethylene, polypropylene, cyclic polyolefin, polyisobutylene, polyethylene terephthalate, unsaturated polyester, fluorine-containing resin, polyvinyl chloride, polyvinylidene chloride, polyvinyl acetate, polyvinyl alcohol, polyvinyl acetal, acrylic resin, polyacrylonitrile, polystyrene, acetal resin, polycarbonate, polyamide, phenol resin, urea resin, epoxy resin, melamine resin, styrene/acrylonitrile copolymer, acrylonitrile/butadiene/styrene copolymer, polyphenylene oxide, and polysulfone. The shape of the carrier is also not particularly limited, but is preferably plate-like.

As the carrier, a carrier having a carbon layer and a chemical modification group on a surface of the carrier is preferably used. The carrier having a carbon layer and a chemical modification group on a surface of the carrier encompasses a carrier having a carbon layer and a chemical modification group on a surface of a substrate, and a carrier having a chemical modification group on a surface of a substrate consisting of a carbon layer. As a material of the substrate, a material known in the art can be used without particular limitation, and the same materials as the materials listed above as the carrier material can be used.

In a microarray, a carrier having a fine plate-like structure is preferably used. The shape is, for example, rectangular, square, or round without limitation, but usually, a 1 to 75 mm square carrier, preferably a 1 to 10 mm square carrier, and more preferably a 3 to 5 mm square carrier is used. It is easy to produce a carrier having a fine plate-like structure, and thus, it is preferable to use a substrate consisting of a silicon material or a resin material. In particular, a carrier having a carbon layer and a chemical modification group on a surface of a substrate consisting of single crystal silicon is more preferable. Single crystal silicon encompasses: single crystal silicon whose crystal axis direction is slightly changed in some portions (referred to as a mosaic crystal in some cases); and single crystal silicon comprising disorder (lattice defect) on an atomic scale.

The carbon layer formed on the substrate is not particularly limited, but it is preferable to use any of synthetic diamond, high-pressure synthetic diamond, natural diamond, soft diamond (for example, diamond-like carbon), amorphous carbon, a carbonaceous substance (for example, graphite, fullerene, or a carbon nanotube), a mixture thereof, or a laminate thereof. Alternatively, a carbide such as hafnium carbide, niobium carbide, silicon carbide, tantalum carbide, thorium carbide, titanium carbide, uranium carbide, tungsten carbide, zirconium carbide, molybdenum carbide, chromium carbide, or vanadium carbide may be used. Here, soft diamond is a general term for an incomplete diamond structure which is a mixture of diamond and carbon, such as what is called diamond-like carbon (DLC), and the mixing ratio thereof is not particularly limited. The carbon layer is advantageous in that the carbon layer has excellent chemical stability, and can withstand a reaction in subsequent introduction of a chemical modification group and binding to a substance to be analyzed, that the binding to a substance to be analyzed is through electrostatic bonding, and thus, is flexible, that the carbon layer is free from UV absorption, and thus, is transparent to UV in a detection system, and that the carbon layer can be electrified during electroblotting. Additionally, the carbon layer is advantageous also in that non-specific adsorption is small in a binding reaction with a substance to be analyzed. As described above, a carrier whose substrate itself consists of a carbon layer may be used.

The carbon layer can be formed using a known method. Examples thereof include a microwave plasma CVD (Chemical vapor deposit) method, an ECRCVD (Electric cyclotron resonance chemical vapor deposit) method, an ICP (Inductive coupled plasma) method, a direct current sputtering method, an ECR (Electric cyclotron resonance) sputtering method, an ionized vapor deposition method, an arc vapor deposition method, a laser vapor deposition method, an EB (Electron beam) vapor deposition method, and a resistance heating vapor deposition method.

In a high-frequency plasma CVD method, a source gas (methane) is decomposed by glow discharge generated between electrodes by a high frequency to synthesize a carbon layer on a substrate. In an ionized vapor deposition method, a source gas (benzene) is decomposed and ionized using thermal electrons generated by a tungsten filament to form a carbon layer on a substrate by a bias voltage. A carbon layer may be formed by an ionized vapor deposition method in a gas mixture consisting of 1 to 99% by volume of hydrogen gas and the balance of 99 to 1% by volume of methane gas.

In an arc vapor deposition method, an arc discharge is generated in a vacuum by applying a direct current voltage between a solid graphite material (cathode evaporation source) and a vacuum vessel (anode) to generate a plasma of carbon atoms from the cathode, and a bias voltage more negative than at the evaporation source is applied to a substrate, whereby carbon ions in the plasma can be accelerated toward the substrate to form a carbon layer.

In a laser vapor deposition method, a carbon layer can be formed by irradiating, for example, Nd:YAG laser (pulse oscillation) light onto a target graphite plate to melt the graphite, and depositing carbon atoms on a glass substrate.

When a carbon layer is formed on a surface of a substrate, the thickness of the carbon layer is usually monomolecular to approximately 100 µm. If the thickness is too small, a surface of a base substrate may be locally exposed. Conversely, if the thickness is too large, productivity deteriorates. Accordingly, the thickness is preferably 2 nm to 1 µm, and more preferably 5 nm to 500 nm.

By introducing a chemical modification group to a surface of a substrate on which a carbon layer is formed, a probe can be strongly immobilized onto a carrier. The chemical modification group to be introduced can be suitably selected by those skilled in the art, and is not particularly limited. Examples thereof include an amino group, carboxyl group, epoxy group, formyl group, hydroxyl group, and active ester group.

An amino group can be introduced, for example, by irradiating a carbon layer with ultraviolet rays in an ammonia gas or by plasma treatment. Alternatively, an amino group can be introduced by chlorinating a carbon layer by irradiating ultraviolet rays in a chlorine gas, and further irradiating ultraviolet rays in an ammonia gas. Alternatively, an amino group can be introduced by allowing a polyvalent amine gas such as methylenediamine or ethylenediamine to react with a chlorinated carbon layer.

A carboxyl group can be introduced, for example, by allowing a suitable compound to react with a carbon layer aminated as described above. Examples of a compound used to introduce a carboxyl group include: a halocarboxylic acid represented by a formula: X-R1-COOH (wherein X represents a halogen atom, and R1 represents a C₁₀₋₁₂ divalent hydrocarbon group), such as chloroacetic acid, fluoroacetic acid, bromoacetic acid, iodoacetic acid, 2-chloropropionic acid, 3-chloropropionic acid, 3-chloroacrylic acid, and 4-chlorobenzoic acid; a dicarboxylic acid represented by a formula: HOOC-R2-COOH (wherein R2 represents a single bond or a C₁₋₁₂ divalent hydrocarbon group), such as oxalic acid, malonic acid, succinic acid, maleic acid, fumaric acid, and phthalic acid; a polyvalent carboxylic acid such as polyacrylic acid, polymethacrylic acid, trimellitic acid, and butanetetracarboxylic acid; a keto acid or an aldehyde acid represented by a formula: R3-CO-R4-COOH (wherein R3 represents a hydrogen atom or a C₁₋₁₂ divalent hydrocarbon group, and R4 represents a C₁₋₁₂ divalent hydrocarbon group); and a monohalide of a dicarboxylic acid, represented by a formula: X-OC-R5-COOH (wherein X represents a halogen atom, and R5 represents a single bond or a divalent C₁₋₁₂ hydrocarbon group), such as succinic acid monochloride and malonic acid monochloride; and acid anhydrides such as phthalic anhydride, succinic anhydride, oxalic anhydride, maleic anhydride, and butanetetracarboxylic anhydride.

An epoxy group can be introduced, for example, by allowing a suitable polyvalent epoxy compound to react with a carbon layer aminated as described above. Alternatively, an epoxy group can be obtained by allowing an organic peracid to react with a carbon-carbon double bond comprised in a carbon layer. Examples of the organic peracid include peracetic acid, perbenzoic acid, diperoxyphthalic acid, performic acid, and trifluoroperacetic acid.

A formyl group can be introduced, for example, by allowing glutaraldehyde to react with a carbon layer aminated as described above.

A hydroxyl group can be introduced, for example, by allowing water to react with a carbon layer chlorinated as described above.

An active ester group refers to an ester group having a highly acidic electron-withdrawing group on the alcohol side of the ester group to activate a nucleophilic reaction, that is, an ester group having high reaction activity. The active ester group is an ester group that has an electron-withdrawing group on the alcohol side of the ester group, and is more activated than an alkyl ester. The active ester group has reactivity to groups such as an amino group, thiol group, and hydroxyl group. More specifically, phenol esters, thiophenol esters, N-hydroxyamine esters, cyanomethyl esters, heterocyclic hydroxy compound esters, and the like are known as active ester groups having a much higher activity than alkyl esters and the like. More specifically, examples of an active ester group include a p-nitrophenyl group, N-hydroxysuccinimide group, succinimide group, phthalimide group, and 5-norbornene-2,3-dicarboximide group. In particular, an N-hydroxysuccinimide group is preferably used.

An active ester group can be introduced, for example, by active esterification of the above-described introduced carboxyl group with an agent for dehydrating condensation such as cyanamide or carbodiimide (for example, 1-[3-(dimethylamino)propyl]-3-ethylcarbodiimide), and a compound such as N-hydroxysuccinimide. By this treatment, a group in which an active ester group such as an N-hydroxysuccinimide group is bound to an end of a hydrocarbon group via an amide bond can be formed (JP2001-139532A).

A wild-type probe or a mutant-type probe is dissolved in a spotting buffer to prepare a spotting solution. The spotting solution is dispensed into a 96-well or 384-well plastic plate. By spotting the dispensed solution onto a carrier using a spotter device or the like, a microarray in which the wild-type probe and the mutant-type probe are immobilized on the carrier can be produced. Alternatively, the spotting solution may be spotted manually using a micropipettor.

After spotting, incubation is preferably performed in order to advance a reaction in which the wild-type probe and the mutant-type probe bind to the carrier. Incubation is usually performed at a temperature of -20 to 100°C, preferably 0 to 90°C, usually for 0.5 to 16 hours, preferably 1 to 2 hours. Incubation is desirably performed in a high-humidity atmosphere, for example, under conditions of 50 to 90% humidity. Following the incubation, washing is preferably performed using a washing solution (for example, 50 mM TBS/0.05% Tween 20, 2×SSC/0.2% SDS solution, ultrapure water, or the like) in order to remove DNA not bound to the carrier.

### 3) Blocking nucleic acid

A blocking nucleic acid is used to prevent a nucleic acid fragment corresponding to a wild type, among nucleic acid fragments amplified using the above-described primer set, from non-specifically hybridizing to a mutant-type probe. The blocking nucleic acid has a base sequence that specifically hybridizes to the nucleic acid fragment corresponding to the wild type. The blocking nucleic acid can be designed based on the base sequence of the nucleic acid fragment amplified using the above-described primer set. Specifically, among the nucleic acid fragments amplified using the above-described primer set, the blocking nucleic acid is designed to be a base sequence complementary to a region comprising at least a position of a gene mutation (wild type).

The blocking nucleic acid is preliminarily mixed with a reaction liquid comprising a wild-type nucleic acid fragment and a mutant-type nucleic acid fragment. The resultant liquid mixture is brought into contact with the above-described DNA chip, whereby a hybridization reaction between the mutant-type nucleic acid fragment and the mutant-type probe can be advanced. Alternatively, a reaction liquid comprising the wild-type nucleic acid fragment and the mutant-type nucleic acid fragment, and a solution comprising the blocking nucleic acid may be mixed on the DNA chip to simultaneously advance specific hybridization between the mutant-type nucleic acid fragment and the mutant-type probe. In any of these cases, the blocking nucleic acid specifically hybridizes to the wild-type nucleic acid fragment, whereby the wild-type nucleic acid fragment can be prevented from non-specifically hybridizing to the mutant-type probe.

In the blocking nucleic acid, the position of a gene mutation (wild type) to be detected is not particularly limited. Additionally, the length of the blocking nucleic acid is not particularly limited, but is preferably 60% or more of the base length of the mutant-type probe. Additionally, the length of the blocking nucleic acid is preferably 140% or less of the base length of the mutant-type probe. For example, when the length of the mutant-type probe is 25 bases in length, the base length of the blocking nucleic acid is preferably 15 to 35 bases in length.

Furthermore, as long as the blocking nucleic acid has a base complementary to the wild-type base at the site of the gene mutation to be detected, the blocking nucleic acid may comprise a base mismatched (noncomplementary) to the wild-type nucleic acid fragment in other regions. When the blocking nucleic acid is 15 bases in length, the number of mismatched bases may be 1 to 3, and is preferably 1 to 2. Additionally, when the blocking nucleic acid is 25 bases in length, the number of mismatched bases may be 1 to 5, and is preferably 1 to 4.

Note that the blocking nucleic acid does not need to be used if non-specific hybridization between the wild-type nucleic acid fragment and the mutant-type probe is slight. The blocking nucleic acid is unnecessary if non-specific hybridization between the wild-type nucleic acid fragment and the mutant-type probe is at a level that does not pose a problem. When the blocking nucleic acid is used, the concentration of the blocking nucleic acid is not particularly limited. For example, the concentration may be suitably set according to the concentration of a non-target nucleic acid and/or the concentration of a target nucleic acid, or according to the concentration of a primer.

More specifically, as blocking nucleic acids, a blocking nucleic acid for a MYD88 gene mutation that is effective in detecting the above-described gene mutation in the MYD88 gene, and a blocking nucleic acid for a CD79B gene mutation that is effective in detecting the above-described gene mutation in the CD79B gene can be designed. For example, in the case of using the above-described set of the forward primer for the MYD88 gene consisting of the base sequence of SEQ ID NO: 79 (5'-CAGGTGCCCATCAGAAG-3') and the reverse primer for the MYD88 gene consisting of the base sequence of SEQ ID NO: 78 (5'-CGCAGACAGTGATGAACC-3'), the base sequence of SEQ ID NO: 89 can be designed as the blocking nucleic acid for a MYD88 gene mutation. Additionally, in the case of using the above-described set of the forward primer for the CD79B gene consisting of the base sequence of SEQ ID NO: 85 (5'-CAGGATGACAGCAAGGC-3') and the reverse primer for the CD79B gene consisting of the base sequence of SEQ ID NO: 86 (5'-CAACCACACCAGCAGATAG-3'), the base sequence of SEQ ID NO: 95 can be designed as the blocking nucleic acid for a CD79B gene mutation.

In particular, in the case of using the above-described set of the forward primer for the MYD88 gene consisting of the base sequence of SEQ ID NO: 79 (5'-CAGGTGCCCATCAGAAG-3') and the reverse primer for the MYD88 gene consisting of the base sequence of SEQ ID NO: 78 (5'-CGCAGACAGTGATGAACC-3'), non-specific hybridization between an amplified wild-type nucleic acid fragment and the MYD88 gene mutant-type probe consisting of the base sequence of SEQ ID NO: 6 (5'-GAAGCGACCGATCCC-3') is slight, and a blocking nucleic acid cannot be said to be necessarily required. That is, the blocking nucleic acid for a MYD88 gene mutation does not need to be used (the blocking nucleic acid concentration is 0 nM). However, in order to more precisely detect a mutant-type nucleic acid fragment, it is preferable to use the blocking nucleic acid for a MYD88 gene mutation consisting of the base sequence of SEQ ID NO: 89. In this case, the concentration of the blocking nucleic acid for a MYD88 gene mutation is not particularly limited, but may be 0 to 500 nM, is preferably 10 to 450 nM, preferably 20 to 400 nM, preferably 30 to 350 nM, preferably 40 to 300 nM, preferably 50 to 250 nM, preferably 60 to 200 nM, and preferably 70 to 150 nM. As an example, the blocking nucleic acid for a MYD88 gene mutation may be used at a concentration of 125 nM.

Additionally, in the case of using the above-described set of the forward primer for the CD79B gene consisting of the base sequence of SEQ ID NO: 85 (5'-CAGGATGACAGCAAGGC-3') and the reverse primer for the CD79B gene consisting of the base sequence of SEQ ID NO: 86 (5'-CAACCACACCAGCAGATAG-3'), non-specific hybridization occurs between an amplified wild-type nucleic acid fragment and a CD79B gene mutant-type probe shown in Table 3. Accordingly, it is preferable to use a blocking nucleic acid for a CD79B gene mutation consisting of the base sequence of SEQ ID NO: 95. In this case, the concentration of the blocking nucleic acid for a CD79B gene mutation is not particularly limited, but may be 125 to 1000 nM, and is preferably 500 to 750 nM. As an example, the blocking nucleic acid for a CD79B gene mutation may be used at a concentration of 750 nM.

### 4) Method of testing gene mutation

As described above, the gene mutation evaluation kit identifies a gene mutation in the CD79B gene and a gene mutation in the MYD88 gene, and can be utilized for diagnosing a disease associated with these gene mutations. Note that, specifically, when these gene mutations are detected, the disease can be classified as the MCD subtype in diffuse large B-cell lymphoma (DLBCL). In this regard, Cancer Cell 39, 1643-1653, December 13, 2021 suggests that, when a disease is classified as the MCD subtype in DLBCL, R-CHOP therapy (rituximab, cyclophosphamide, doxorubicin, vincristine, and prednisone) combined with ibrutinib, which is a kind of BTK inhibitor, is effective. Accordingly, by identifying the MCD type of DLBCL using the above-described gene mutation evaluation kit, useful information for determining a treatment policy can be provided.

Below, a procedure for identifying a gene mutation in the MYD88 gene and a gene mutation in the CD79B gene using the gene mutation evaluation kit will be specifically described. In the following description, a mode using a microarray having a wild-type probe and a mutant-type probe related to a gene mutation in the MYD88 gene, and a wild-type probe and a mutant-type probe related to a gene mutation in the CD79B gene is adopted.

By using the above-described microarray, a gene mutation in the MYD88 gene and a gene mutation in the CD79B gene, for example, in a diagnosis subject can be identified.

When a gene mutation is specifically tested, the test comprises: a step of extracting DNA from a sample derived from a diagnosis subject; a step of amplifying a region comprising a gene mutation to be tested, using extracted DNA as a template; a step of hybridizing an amplified nucleic acid fragment with a wild-type probe and a mutant-type probe using the above-described microarray; and a step of detecting signals from the wild-type probe and the mutant-type probe. In the above hybridization step, "wild-type probe and mutant-type probe" is meant to comprise: a wild-type probe and a mutant-type probe related to a gene mutation in the MYD88 gene; and a wild-type probe and a mutant-type probe related to a gene mutation in the CD79B gene. Similarly, "wild-type probe and mutant-type probe" in the step of detecting signals is meant to comprise: a wild-type probe and a mutant-type probe related to a gene mutation in the MYD88 gene; and a wild-type probe and a mutant-type probe related to a gene mutation in the CD79B gene.

The diagnosis subject is usually a human. Although there is no particular limitation on the race or the like, the subject is particularly Asian, preferably East Asian, and particularly preferably Japanese. Additionally, a diagnosis subject can be a patient suspected of having a disease associated with a gene mutation to be tested. A sample derived from a diagnosis subject is not particularly limited. Examples thereof include blood-related samples (blood, serum, plasma, and the like), lymph fluid, feces, cancer cells, and crushed materials and extracts of tissues or organs.

First, DNA is extracted from a sample collected from a diagnosis subject. An extraction means is not particularly limited. For example, a DNA extraction method using phenol/chloroform, ethanol, sodium hydroxide, CTAB, or the like can be used.

Next, an amplification reaction is performed using the obtained DNA as a template to amplify a region comprising a gene mutation to be tested. As the amplification reaction, a polymerase chain reaction (PCR), LAMP (Loop-Mediated Isothermal Amplification), ICAN (Isothermal and Chimeric primer-initiated Amplification of Nucleic acids) method, or the like can be applied. Note that, as described above, by labeling a primer used in a nucleic acid amplification reaction, an amplified nucleic acid fragment can be labeled. That is, by adding a labeling substance to any one of a forward primer and a reverse primer comprised in a primer set for a CD79B gene mutation, and to any one of a forward primer and a reverse primer comprised in a primer set for a MYD88 gene mutation, the amplified nucleic acid fragment can be labeled.

This reaction system is a reaction system comprising: an agent for buffering necessary for nucleic acid amplification and labeling; a heat-resistant DNA polymerase; a primer set for a MYD88 gene mutation and a primer set for a CD79B gene mutation; labeled nucleotide triphosphates (specifically, nucleotide triphosphates to which a fluorescent label or the like is added); nucleotide triphosphates; magnesium chloride; and the like.

Additionally, an amplified nucleic acid fragment depends on the design of a primer set for a MYD88 gene mutation and a primer set for a CD79B gene mutation, and is, for example, preferably 1 kbp or less, more preferably 800 bp or less, still more preferably 500 bp or less, and particularly preferably 350 bp or less.

A hybridization reaction is performed between an amplified nucleic acid obtained as described above and both a wild-type probe and a mutant-type probe immobilized on a carrier. Signals from the wild-type probe and the mutant-type probe are detected based on a label of the hybridized nucleic acid fragment, whereby the gene mutation in a diagnosis subject can be tested.

For example, when a fluorescent label is used, a signal from a label can be digitized into a signal intensity by detecting a fluorescence signal using a fluorescence scanner and analyzing the detected fluorescence signal with image analysis software. A hybridization reaction is preferably performed under stringent conditions. Stringent conditions refer to conditions under which specific hybrids are formed and non-specific hybrids are not formed. For example, the stringent conditions refer to conditions in which a hybridization reaction is performed at 47 to 52°C (specifically 51.5°C) for 15 to 90 minutes (specifically 30 minutes), followed by washing 30 times under conditions of 0.1×SSC/0.1% SDS and 25°C, and then washing 80 times under conditions of 1×SSC and 25°C. Note that, when the chain length of the probe is short, the hybridizing temperature is more preferably lower than this, and that, when the chain length is long, the hybridizing temperature is more preferably higher than this. It goes without saying that the hybridizing temperature having specificity becomes higher as the salt concentration becomes higher, and conversely, the hybridizing temperature having specificity becomes lower as the salt concentration becomes lower.

Additionally, when a microarray comprising a mutant-type probe and a wild-type probe related to the above-described gene mutation is used, the gene mutation can be tested using signal intensities from the mutant-type probe and the wild-type probe. Specifically, signal intensities from a mutant-type probe and a wild-type probe are respectively measured, and a value for determination for evaluating a signal intensity derived from the mutant-type probe is calculated. Examples of calculating a value for determination include a method of evaluating a mutant type corresponding to a mutant-type probe, using Formula 1: [Signal intensity derived from mutant-type probe]/(Signal intensity derived from wild-type probe] + [Signal intensity derived from mutant-type probe]).

A value for determination calculated by Formula 1 above is compared with a predetermined threshold value (cut-off value). If the value for determination exceeds the threshold value, it is determined that an amplified nucleic acid having a gene mutation (mutant type) corresponding to a mutant-type probe is comprised. If the value for determination falls below the threshold value, it is determined that an amplified nucleic acid does not comprise the gene mutation (mutant type).

Here, the threshold value is not particularly limited, but can be defined, for example, based on a value for determination calculated by Formula 1 above using a specimen whose gene mutation is confirmed to be a wild type. More specifically, a plurality of values for determination are calculated using a plurality of specimens whose gene mutations are confirmed to be wild types, and the average of the values + 3σ (σ: standard deviation) can be used as a threshold value. Note that an average value + 2σ or an average value + σ can also be used as a threshold value.

As described above, by utilizing a microarray comprising a mutant-type probe and a wild-type probe related to each of a gene mutation in the MYD88 gene and a gene mutation in the CD79B gene, whether the gene mutation is a mutant type or a wild type can be identified with high precision.

In particular, in the case of using the above-described primer set for a MYD88 gene mutation consisting of a forward primer for the MYD88 gene consisting of the base sequence of SEQ ID NO: 79 (5'-CAGGTGCCCATCAGAAG-3') and a reverse primer for the MYD88 gene consisting of the base sequence of SEQ ID NO: 78 (5'-CGCAGACAGTGATGAACC-3') and the above-described primer set for a CD79B gene mutation consisting of a forward primer for the CD79B gene consisting of the base sequence of SEQ ID NO: 85 (5'-CAGGATGACAGCAAGGC-3') and a reverse primer for the CD79B gene consisting of the base sequence of SEQ ID NO: 86 (5'-CAACCACACCAGCAGATAG-3'), target nucleic acid fragments can each be obtained stably and in large amplification amounts. Additionally, non-specific amplification based on non-specific hybridization of a forward primer and/or a reverse primer can be kept low. As a result, by using these primer sets, each gene mutation can be identified with better sensitivity.

Additionally, regarding a nucleic acid fragment obtained using the above-described set of the forward primer for the MYD88 gene consisting of the base sequence of SEQ ID NO: 79 (5'-CAGGTGCCCATCAGAAG-3') and the reverse primer for the MYD88 gene consisting of the base sequence of SEQ ID NO: 78 (5'-CGCAGACAGTGATGAACC-3'), a gene mutation in the MYD88 gene in a diagnosis subject can be identified with high precision by using a MYD88 gene wild-type probe consisting of the base sequence of SEQ ID NO: 2 (5'-AGCGACTGATCCCCAT-3') and a MYD88 gene mutant-type probe consisting of the base sequence of SEQ ID NO: 6 (5'-GAAGCGACCGATCCC-3').

Incidentally, regarding a gene mutation in the CD79B gene, a first probe that is a mutant-type probe for detecting missense mutations (M1 to M6) and nonsense mutations (M7 and M8), and a second probe that is a mutant-type probe for detecting a silent mutation (M9) are designed for a nucleic acid fragment obtained using the above-described set of the forward primer for the CD79B gene consisting of the base sequence of SEQ ID NO: 85 (5'-CAGGATGACAGCAAGGC-3') and the reverse primer for the CD79B gene consisting of the base sequence of SEQ ID NO: 86 (5'-CAACCACACCAGCAGATAG-3').

In this case, whether a gene mutation in the CD79B gene is a wild type or a mutant type can be identified as follows. In particular, in the present invention, when the second probe detects that the above-described gene mutation is a silent mutation, the gene mutation can be determined to be a wild type.

Specifically, when a microarray comprising a first probe, a second probe, and a wild-type probe is used, a gene mutation in the CD79B gene can be tested using signal intensities from the first probe, the second probe, and the wild-type probe. That is, signal intensities from the first probe, the second probe, and the wild-type probe are respectively measured, and a value for determination for evaluating a signal intensity derived from the first probe or the second probe is calculated. Examples of calculating a value for determination include a method of evaluating a missense mutation and/or a nonsense mutation corresponding to a first probe, using Formula 1: [Signal intensity derived from first probe]/([Signal intensity derived from wild-type probe] + [Signal intensity derived from first probe]), and evaluating a silent mutation corresponding to a second probe, using Formula 2: [Signal intensity derived from second probe]/([Signal intensity derived from wild-type probe] + [Signal intensity derived from second probe]).

Then, a value for determination calculated by Formula 1 above is compared with a predetermined threshold value (cut-off value). If the value for determination exceeds the threshold value, it is determined that an amplified nucleic acid comprises a missense mutation and/or a nonsense mutation corresponding to the first probe related to the gene mutation. If the value for determination falls below the threshold value, it is determined that an amplified nucleic acid does not comprise the missense mutation and/or the nonsense mutation. Additionally, a value for determination calculated by Formula 2 above is compared with a predetermined threshold value (cut-off value). If the value for determination exceeds the threshold value, it is determined that an amplified nucleic acid comprises a silent mutation corresponding to the second probe related to the gene mutation. If the value for determination falls below the threshold value, it is determined that an amplified nucleic acid does not comprise the silent mutation.

When it is determined that a silent mutation is comprised, based on Formula 2 above, it is determined that a gene mutation in the CD79B gene to be tested is a wild type. Additionally, also when it is determined that a missense mutation and/or a nonsense mutation are not comprised, based on Formula 1 above, and a silent mutation is not comprised, based on Formula 2 above, it is determined that a gene mutation to be tested is a wild type. Thus, by utilizing a microarray comprising a first probe, a second probe, and a wild-type probe for testing a gene mutation in the CD79B gene, whether the gene mutation is a mutant type of a missense mutation and/or a nonsense mutation, or a wild type, can be identified with high precision.

As described above, by identifying, for a diagnosis subject, whether a gene mutation in the MYD88 gene is a wild type or a mutant type, and whether a gene mutation in the CD79B gene is a wild type or a mutant type, effective information for a diagnosis of the MCD type of diffuse large B-cell lymphoma (DLBCL) can be provided.

### Examples

The present invention will be described in more detail below with reference to an Example, but the technical scope of the present invention is not limited to the following Example.

### <Sequence design>

In this Example, regarding a gene mutation in the MYD88 gene and a gene mutation in the CD79B gene, a primer set for amplifying a region comprising the gene mutation, a probe for detecting a wild type or a mutant type comprised in a nucleic acid fragment amplified by the primer set, and a blocking nucleic acid corresponding to a wild type in a gene mutation in the MYD88 gene and a gene mutation in the CD79B gene were designed.

The probes corresponding to gene mutations in the MYD88 gene, which probes were designed in this Example, are shown in Table 4. Note that, in the column "Wild-type (W)/Mutant type (M)" in Table 4, W denotes a probe corresponding to a wild type, and M denotes a probe corresponding to a mutant type.

**[Table 4]**

| Wild-type (W)/ Mutant type (M) | Name | Base sequence [5'→3'] | SEQ ID NO |
|---|---|---|---|
| W | MYD88_W_v1 | CAGAAGCGACTGATCCCCATC | 1 |
| W | MYD88_W_v2-1 | AGCGACTGATCCCCAT | 2 |
| W | MYD88_W_v2-2 | AAGCGACTGATCCCCAT | 3 |
| W | MYD88_W_v2-3 | CAGAAGCGACTGATCCC | 4 |
| M | MYD88_M_v1 | CAGAAGCGACCGATCCCCATCA | 5 |
| M | MYD88_M_v2-1 | GAAGCGACCGATCCC | 6 |
| M | MYD88_M_v2-2 | CAGAAGCGACCGATCC | 7 |

The probes corresponding to genes mutation in the CD79B gene, designed in this Example, are shown in Tables 5 and 6. Note that, in the column "Wild-type (W)/Mutant type (M)" in Tables 5 and 6, W denotes a probe corresponding to a wild type, and M1 to M9 denote a probe corresponding to a mutant type. The notation of M1 to M9 is in accordance with Table 3.

**[Table 5]**

| Wild-type (W)/ Mutant type (M) | Name | Base sequence [5'→3'] | SEQ ID NO |
|---|---|---|---|
| W | CD79B_W_v1 | TCCTTACCTCGTAGGTGTGATCTTC | 8 |
| W | CD79B_W_v2 | CTTACCTCGTAGGTGTGATC | 9 |
| W | CD79B_W_v3-1 | CTTACCTCGTAGGTGTGAT | 10 |
| W | CD79B_W_v3-2 | TTACCTCGTAGGTGTGATC | 11 |
| M1 | CD79B_M1_v1 | TCTCCTTACCTCGTGGGTGTGATCTTC | 12 |
| M1 | CD79B_M1_v2-1 | CTTACCTCGTGGGTGTG | 13 |
| M1 | CD79B_M1_v2-2 | CTTACCTCGTGGGTGTGA | 14 |
| M2 | CD79B_M2_v1 | TCTCCTTACCTCGTTGGTGTGATCTTC | 15 |
| M2 | CD79B_M2_v2-1 | **C**TTACCTCGTTGGTGTGATC | 16 |
| M2 | CD79B_M2_v2-2 | CCTTACCTCGTTGGTGTGATC | 17 |
| M2 | CD79B_M2_v2-3 | CCTTACCTCGTTGGTGTGAT | 18 |
| M2 | CD79B_M2_v3-1 | CTTACCTCGTTGGTGTGA | 19 |
| M2 | CD79B_M2_v3-2 | TCTCCTTACCTCGTTGGTGT | 20 |
| M2 | CD79B_M2_v3-3 | CCTCGTTGGTGTGATCTT | 21 |
| M3 | CD79B_M3_v1 | TCTCCTTACCTCGTCGGTGTGATCTTC | 22 |
| M3 | CD79B_M3_v2-1 | CTTACCTCGTCGGTGTG | 23 |
| M3 | CD79B_M3_v2-2 | CTTACCTCGTCGGTGTGA | 24 |
| M4 | CD79B_M4_v1 | TCTCCTTACCTCGGAGGTGTGATCTTC | 25 |
| M4 | CD79B_M4_v2-1 | CTTACCTCGGAGGTGTG | 26 |
| M4 | CD79B_M4_v2-2 | CTTACCTCGGAGGTGTGA | 27 |
| M4 | CD79B_M4_v3-1 | CTTACCTCGGAGGTGT | 28 |
| M4 | CD79B_M4_v3-2 | CCTCGGAGGTGTGATC | 29 |
| M4 | CD79B_M4_v4-1 | TTACCTCGGAGGTGTG | 30 |
| M4 | CD79B_M4_v4-2 | TTACCTCGGAGGTGT | 31 |
| M4 | CD79B_M4_v4-3 | TTACCTCGGAGGTGTGAT | 32 |
| M4 | CD79B_M4_v4-4 | CTCGGAGGTGTGATCTT | 33 |
| M5 | CD79B_M5_v1 | TCTCCTTACCTCGCAGGTGTGATCTTC | 34 |
| M5 | CD79B_M5_v2 | CTTACCTCGCAGGTGTG | 35 |
| M5 | CD79B_M5_v5-1 | CCTCGCAGGTGTGATC | 36 |
| M5 | CD79B_M5_v5-2 | TTACCTCGCAGGTGTGAT | 37 |
| M5 | CD79B_M5_v5-3 | CTCGCAGGTGTGATCTT | 38 |

**[Table 6]**

| Wild-type (W)/ Mutant type (M) | Name | Base sequence [5'→3'] | SEQ ID NO |
|---|---|---|---|
| M6 | CD79B_M6_v1 | TCTCCTTACCTCGAAGGTGTGATCTTC | 39 |
| M6 | CD79B_M6_v2-1 | CTTACCTCGAAGGTGTGATC | 40 |
| M6 | CD79B_M6_v2-2 | CTTACCTCGAAGGTGTGAT | 41 |
| M6 | CD79B_M6_v2-3 | CCTTACCTCGAAGGTGTGAT | 42 |
| M6 | CD79B_M6_v3-1 | CTTACCTCGAAGGTGTG | 43 |
| M6 | CD79B_M6_v3-2 | CCTCGAAGGTGTGATC | 44 |
| M6 | CD79B_M6_v4-1 | CCTCGAAGGTGTGAT | 45 |
| M6 | CD79B_M6_v4-2 | TTACCTCGAAGGTGTGAT | 46 |
| M6 | CD79B_M6_v4-3 | CTCGAAGGTGTGATCTTC | 47 |
| M6 | CD79B_M6_v4-4 | CTTACCTCGAAGGTGTGA | 48 |
| M7 | CD79B_M7_v1 | CTCTCCTTACCTCCTAGGTGTGATCTTCC | 49 |
| M7 | CD79B_M7_v2-1 | CTTACCTCCTAGGTGTGATCT | 50 |
| M7 | CD79B_M7_v2-2 | CCTTACCTCCTAGGTGTGA | 51 |
| M8 | CD79B_M8_v1 | CTCTCCTTACCTCTTAGGTGTGATCTTCC | 52 |
| M8 | CD79B_M8_v2-1 | CTTACCTCTTAGGTGTGATCTTC | 53 |
| M8 | CD79B_M8_v2-2 | CCTTACCTCTTAGGTGTGATC | 54 |
| M8 | CD79B_M8_v2-3 | CCTTACCTCTTAGGTGTGATCT | 55 |
| M8 | CD79B_M8_v3-1 | CCTTACCTCTTAGGTGTGATCTT | 56 |
| M8 | CD79B_M8_v3-2 | TCTCCTTACCTCTTAGGTGTG | 57 |
| M8 | CD79B_M8_v3-3 | CCTTACCTCTTAGGTGTG | 58 |
| M8 | CD79B_M8_v3-4 | TCTCCTTACCTCTTAGGT | 59 |
| M8 | CD79B_M8_v3-5 | TCCTTACCTCTTAGGTGT | 60 |
| M8 | CD79B_M8_v3-6 | TTACCTCTTAGGTGTGATC | 61 |
| M8 | CD79B_M8_v4-1 | TTACCTCTTAGGTGTGAT | 62 |
| M8 | CD79B_M8_v4-2 | TTACCTCTTAGGTGTGA | 63 |
| M8 | CD79B_M8_v4-3 | TCCTTACCTCTTAGGTGTG | 64 |
| M8 | CD79B_M8_v4-4 | TCTCCTTACCTCTTAGGTGT | 65 |
| M9 | CD79B_M9_1 | TCTCCTTACCTCATAGGTGTG | 66 |
| M9 | CD79B_M9_2 | TTACCTCATAGGTGTGATC | 67 |
| M9 | CD79B_M9_3 | TCCTTACCTCATAGGTGTG | 68 |

Additionally, the primer set for amplifying a region comprising a gene mutation in the MYD88 gene, designed in this Example, is shown in Table 7.

**[Table 7]**

| Name | Base sequence [5'→3'] | Tm (°C) | SEQ ID NO |
|---|---|---|---|
| MYD_F01 | ATGCCAGCATGGCACCCCTT | 62.8 | 69 |
| MYD_R01 | GCAGGGGTTGGTGTAGTCGCA | 62.1 | 70 |
| MYD88_Fw-2 | GCAAGGGCCTGATG | 55.6 | 71 |
| MYD88_Rv-2-2 | GTGATGAACCTCAGGATG | 56.8 | 72 |
| MYD88_Fw-3 | GGCTTGTCCCACCAT | 57.9 | 73 |
| MYD88_Rv-3 | TCGCAGACAGTGATGAA | 57.9 | 74 |
| MYD88_Fw-4 | CCTTGGCTTGCAGGT | 58.6 | 75 |
| MYD88_Rv-4 | GGTGTAGTCGCAGACAG | 58.6 | 76 |
| MYD88_Fw-5 | CCTTGGCTTGCAGGTG | 60.0 | 77 |
| MYD88_Rv-5 | CGCAGACAGTGATGAACC | 60.1 | 78 |
| MYD88_Fw-5-3 | CAGGTGCCCATCAGAAG | 59 | 79 |
| MYD88_Rv-5-4 | GATGCTGGGGAACTCTTTCTTCATTGC | 59.9 | 80 |

Furthermore, the primer set for amplifying a region comprising a gene mutation in the CD79B gene, designed in this Example, is shown in Table 8.

**[Table 8]**

| Name | Base sequence [5'→3'] | Tm (°C) | SEQ ID NO |
|---|---|---|---|
| CD79B-Fw_1 | ACTCTGATCTCCATCCCTCTC | 62 | 81 |
| CD79B-Rv_1 | CAACCACACCAGCAGATAGT | 62 | 82 |
| CD79B-Fw_3 | CACTCTGATCTCCATCCC | 58.1 | 83 |
| CD79B-Rv_3 | ACCACACCAGCAGATAG | 57.9 | 84 |
| CD79B-Fw_4 | CAGGATGACAGCAAGGC | 59.8 | 85 |
| CD79B-Rv_4 | CAACCACACCAGCAGATAG | 59.9 | 86 |

Furthermore, a blocking nucleic acid corresponding to a wild type in a gene mutation in the MYD88 gene, designed in this Example, is shown in Table 9.

**[Table 9]**

| Name | Base sequence [5'→3'] | SEQ ID NO |
|---|---|---|
| MYD88_B_v1 | GCGACTGATCCCCA | 87 |
| MYD88_B_v2-1 | GCGACTGATCCCCAT | 88 |
| MYD88_B_v2-2 | GACTGATCCCCATC | 89 |
| MYD88_B_v2-3 | AGAAGCGACTGATC | 90 |

Furthermore, a blocking nucleic acid corresponding to a wild type in a gene mutation in the CD79B gene, designed in this Example, is shown in Table 10.

**[Table 10]**

| Name | Base sequence [5'→3'] | SEQ ID NO |
|---|---|---|
| CD79B_B_v1 | ACCTCGTAGGTGTGA | 91 |
| CD79B_B_v2-3 | TTACCTCGTAGGTGT | 92 |
| CD79B_B_v3-3 | CCTTACCTCGTAGGT | 93 |
| CD79B_B_v4-1 | CTTACCTCGTAGGTGTG | 94 |
| CD79B_B_v4-2 | TACCTCGTAGGTGTGA | 95 |
| CD79B_B_v4-3 | TCCTTACCTCGTAGGTG | 96 |

### <Examination of optimal mutant-type probe>

Regarding the probes listed in Tables 4 to 6, fluorescence intensities were measured using a 100% mutation model specimen or a 5% mutation model specimen. Specifically, a DNA chip on which each of the above-described various probes was immobilized was set in a gene analyzer BIOSHOT HT-32 (manufactured by Toyo Kohan Co., Ltd.), and a hybridization reaction with a model specimen was performed. A DNA chip needle having the DNA chip fixed thereon, a washing solution (0.1×SSC/0.1% SDS solution, room temperature) for washing excess reagents after a hybridization reaction of the DNA chip, a rinse solution (1×SSC solution, room temperature), and a detection solution (1×SSC solution, room temperature) for fluorescence detection were set in BIOSHOT HT-32, and an operation was started.

By an automatic operation, the DNA chip needle was inserted in a state where a reaction liquid in a designated PCR tube was heated to 51.5°C, and a hybridization reaction was performed for 30 minutes. After the hybridization reaction, the DNA chip needle was quickly immersed in a washing solution tank, and the solution was stirred 30 times such that the DNA chip moved in and out of the washing solution surface. The DNA chip needle after washing was immersed in a rinse solution tank, and the solution was stirred 80 times such that the DNA chip moved in and out of the rinse solution surface.

The DNA chip needle after rinsing was slowly immersed in a detection solution tank, and excitation light was taken into a CCD camera for 2 seconds by irradiation with a single wavelength laser of 640 nm. Then, a value for determination was calculated from a measured fluorescence intensity value according to the following formula. [Value for determination] = [Fluorescence intensity of mutant-type probe]/([Fluorescence intensity of mutant-type probe] + [Fluorescence intensity of wild-type probe])

Note that a 100% mutation model specimen means a sample in which the ratio of the mutant type to the wild type in a gene mutation to be detected is 100:0. Additionally, a 5% mutation model specimen means a sample in which the ratio of the mutant type to the wild type in a gene mutation to be detected is 5:95.

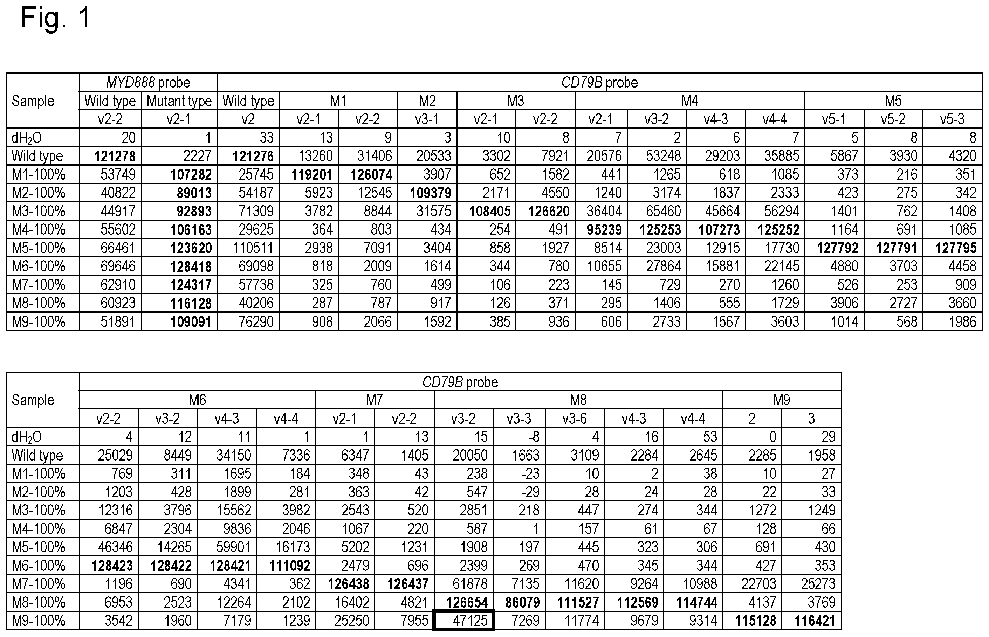

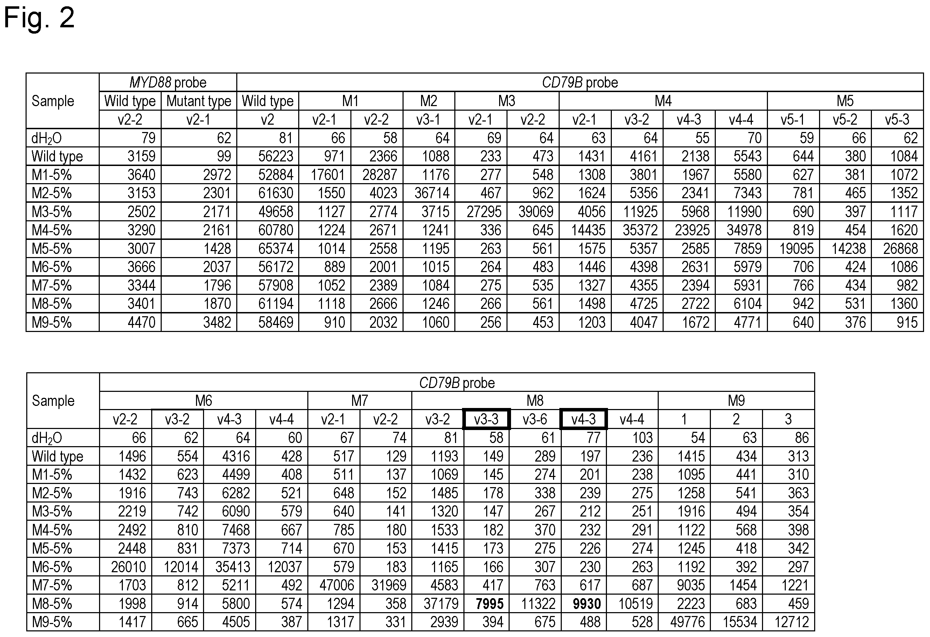

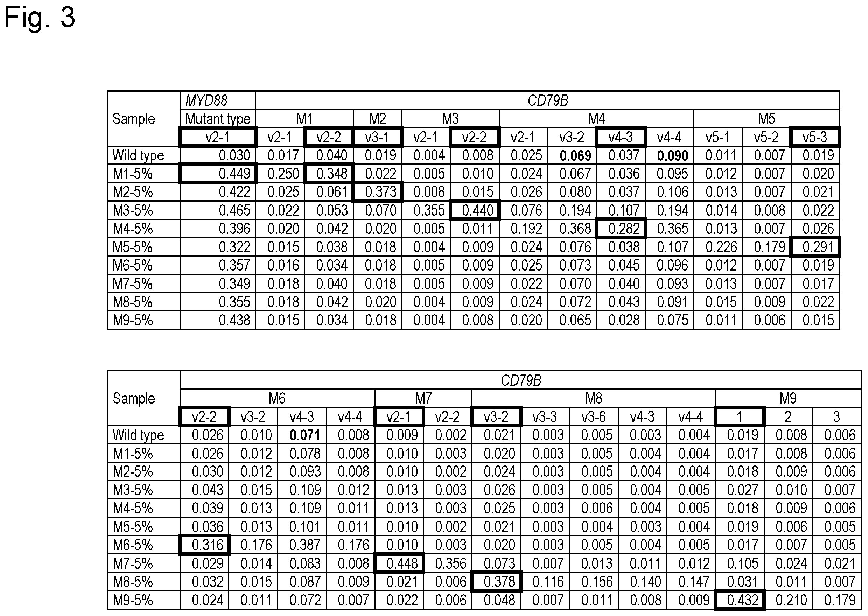

The results of measuring fluorescence intensity values in using a 100% mutation model specimen are shown in Figure 1, the results of measuring fluorescence intensity values in using a 5% mutation model specimen are shown in Figure 2, and the results of measuring values for determination from the results shown in Figure 2 are shown in Figure 3. Note that, in Figures 1 to 3, the results of calculating fluorescence intensity values from the respective probes using a wild-type sample in which the ratio of the mutant type to the wild type in a gene mutation to be detected is 0:100 are also shown for comparison.

Specificities of the mutant-type probe and the wild-type probe were confirmed from fluorescence intensity values shown in Figure 1. The result has revealed that excellent fluorescence intensity values were obtained from all the tested mutant-type probes and wild-type probes. Additionally, for all the tested mutant-type probes and wild-type probes, a cross-reaction was suppressed to approximately 1/2 or less of a specific fluorescence intensity value, and it was determined that a target gene mutation (mutant type and wild type) was able to be distinguished.

Furthermore, according to the results shown in Figure 1, a strong fluorescence intensity value (47125) was detected from an M8 probe (CD79B_M8_v3-2) with a silent mutation 591C>T denoted as M9 among the gene mutations in the CD79B gene. If detection of M9, which is a silent mutation, is not attempted using a mutant-type probe, there is a possibility that a sample having a silent mutation of M9 is erroneously identified as an M8 mutation. Accordingly, a mutant-type probe corresponding to M9, which is a silent mutation, is used to reliably identify the silent mutation in identifying a gene mutation in the CD79B gene, whereby an erroneous determination can be prevented. Additionally, when a silent mutation is identified in this case, the mutation is determined to be a "wild type". This enables precisely identifying whether a gene mutation in the CD79B gene is a mutant type or a wild type, and can provide very effective information on a disease associated with a gene mutation in the CD79B gene.

On the other hand, Figure 2 shows fluorescence intensity values from a mutant-type probe and a wild-type probe in a case where a sample having a low mutation ratio was used. By obtaining a fluorescence intensity value equal to or higher than a desired value even in a sample having a low mutation ratio, a target gene mutation can be detected with high precision. For example, assuming that a fluorescence intensity value of 10000 or more is preferable, the results shown in Figure 2 reveal that sufficient fluorescence intensity values are not obtained from CD79B_M8_v3-3 and CD79B_M8_v4-3.

Furthermore, Figure 3 shows values for determination calculated according to the above-described Formula from fluorescence intensity values shown in Figure 2. As can be understood from the Formula for calculating a value for determination, the higher the value for determination, the better the separation ability by a mutant-type probe and a wild-type probe. Additionally, it can be said that the lower the value for determination calculated for a mutant-type probe used with a wild-type sample, the better the separation ability. For example, based on the criterion that a value for determination calculated for a mutant-type probe used with a wild-type sample is 0.05 or less, the results shown in Figure 3 reveal that none of CD79B_M4_v3-2, CD79B_M4_v4-4, and CD79B_M6-v4-3 have a sufficient separation ability.

As a result of comprehensively considering the above-described results, it has been revealed that, for a gene mutation in the MYD88 gene, MYD88_M_v2-1 is preferably adopted as a mutant-type probe. Additionally, the results have revealed that, for the gene mutations (nine types from M1 to M9) in the CD79B gene, CD79B_M1_v2-2, CD79B_M2_v3-1, CD79B_M3-v2-2, CD79B_M4_v4-3, CD79B_M5_v5-3, CD79B_M6_v2-2, CD79B_M7_v2-1, CD79B_M8_v3-2, and CD79B_M9-1 are preferably adopted.

### <Examination of optimal wild-type probe>

First, a wild-type probe for detecting a wild type in a gene mutation in the MYD88 gene was examined. Fluorescence intensity values and values for determination from a wild-type probe and a mutant-type probe (v2-1) were calculated using tested wild-type samples and a 5% mutation model specimen. Additionally, fluorescence intensity values and values for determination were calculated in the same manner using genomic DNA (gDNA-1) in place of wild-type samples and a 5% mutation model specimen.

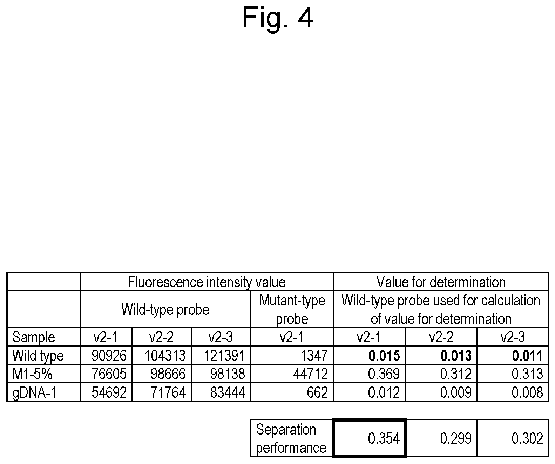

The results are shown in Figure 4. In selecting a wild-type probe, conditions required for the wild-type probe were that the higher fluorescence intensity value is better when genomic DNA is used, that the separation ability is high (that is, a difference between a mutant type value for determination and a wild-type value for determination is large), and that a value for determination calculated by the above-described Formula from a fluorescence intensity value is low when a wild-type sample is used. Specifically, criteria were set such that the fluorescence intensity value from a wild-type probe is 10000 or more when genomic DNA is used, that the separation ability is highest among tested wild-type probes (a difference between a mutant type value for determination and a wild-type value for determination is maximum), and that a value for determination is 0.05 or less when a wild-type sample is used. Based on the criteria, comprehensive evaluations were made. As a result, v2-1, which had a highest separation ability, was successfully selected as an optimal wild-type probe.

Next, a wild-type probe for detecting a wild type in a gene mutation in the CD79B gene was examined. The examination methods and evaluation criteria were in accordance with the above-described methods of selecting a wild-type probe in a gene mutation in the MYD88 gene. As mutant-type probes, CD79B_M1_v2-2, CD79B_M2_v3-1, CD79B_M3-v2-2, CD79B_M4_v4-3, CD79B_M5_v5-3, CD79B_M6_v2-2, CD79B_M7_v2-1, CD79B_M8_v3-2, and CD79B_M9-1 selected above were used.

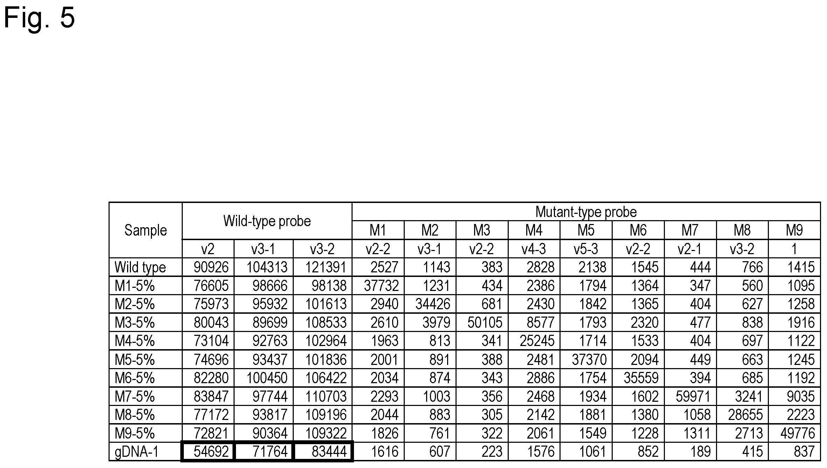

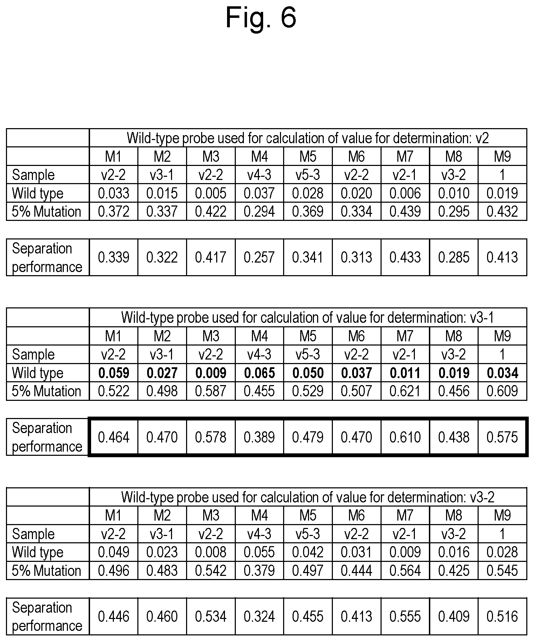

The results of measuring fluorescence intensity values are shown in Figure 5, and the results of calculating values for determination from fluorescence intensity values are shown in Figure 6. As can be seen from Figures 5 and 6, v3-1 was successfully selected as an optimal wild-type probe, comprehensively considering that a fluorescence intensity value is high when genomic DNA is used, that the separation ability is high, and that a value for determination is low when a wild-type sample is used.

### <Performance evaluation of selected mutant-type probe and wild-type probe>

Using a mutant-type probe (MYD88_M_v2-1) and a wild-type probe (MYD88_W_v2-1) selected for a gene mutation in the MYD88 gene, and a mutant-type probe (CD79B_M1_v2-2, CD79B_M2_v3-1, CD79B_M3-v2-2, CD79B_M4_v4-3, CD79B_M5_v5-3, CD79B_M6_v2-2, CD79B_M7_v2-1, CD79B_M8_v3-2, and CD79B_M9-1) and a wild-type probe (CD79B_W_v3-1) selected for a gene mutation in the CD79B gene, the gene mutations in genomic DNAs were identified. Note that, as genomic DNAs, three kinds: gDNA-1, gDNA-2, and gDNA-3 were used. gDNA-1 is genomic DNA derived from a healthy individual. gDNA-2 is genomic DNA derived from a non-Hodgkin lymphoma patient. gDNA-3 is genomic DNA derived from a lymphoplasmacytic lymphoma.

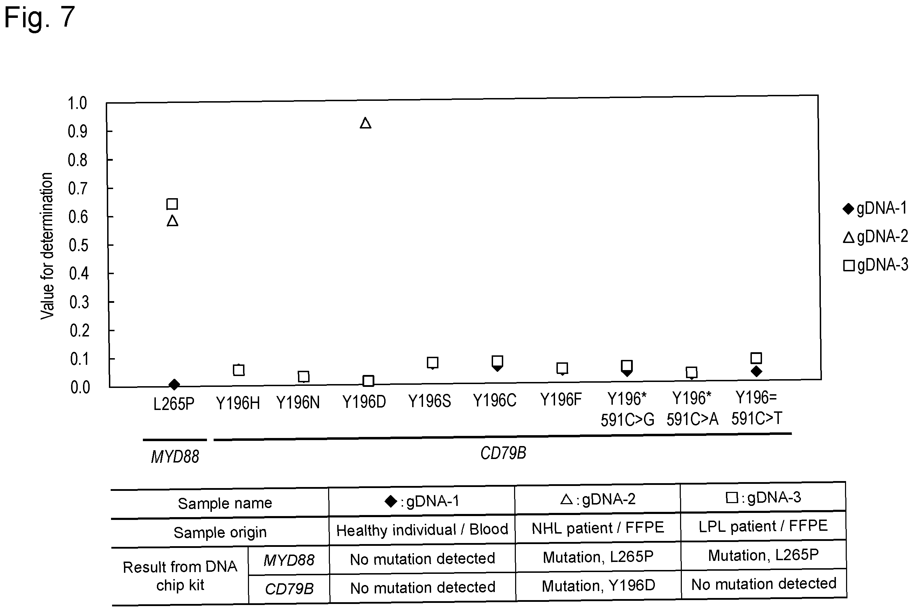

The results of identifying each gene mutation using these mutant-type probes and wild-type probes are shown in Figure 7. As shown in Figure 7, it has been identified regarding gDNA-1 that the gene mutation in the MYD88 gene is not a mutant type, and the gene mutation in the CD79B gene is not a mutant type. Additionally, it has been identified regarding gDNA-2 that the gene mutation in the MYD88 gene is a mutant type (L265P), and the gene mutation in the CD79B gene is the mutant type (Y196D, M3). Additionally, it has been identified regarding gDNA-3 that the gene mutation in the MYD88 gene is a mutant type (L265P), and the gene mutation in the CD79B gene is not a mutant type.

In order to verify the results obtained when the mutant-type probes and the wild-type probe shown in Figure 7 were used, the gene mutation in the MYD88 gene and the gene mutation in the CD79B gene in three kinds of genomic DNAs tested (gDNA-1, gDNA-2, and gDNA-3) were checked by a direct sequencing method. As a result, it has been confirmed that both the gene mutation in the MYD88 gene and the gene mutation in the CD79B gene in gDNA-1 are wild types. Additionally, it has been confirmed that the gene mutation in the MYD88 gene in gDNA-1 is an L265P mutant type, and the gene mutation in the CD79B gene is a Y196D mutant type (M3). Furthermore, it has been confirmed that the gene mutation in the MYD88 gene in gDNA-3 is an L265P mutant type, and the gene mutation in the CD79B gene is a wild type.

The results of identifying each gene mutation using a mutant-type probe and a wild-type probe selected as described above completely matched the results confirmed by the direct sequencing method. This has revealed that the mutant-type probe and the wild-type probe selected as described above can accurately detect each gene mutation with high precision, even in actual use.

### <Design of primer set>

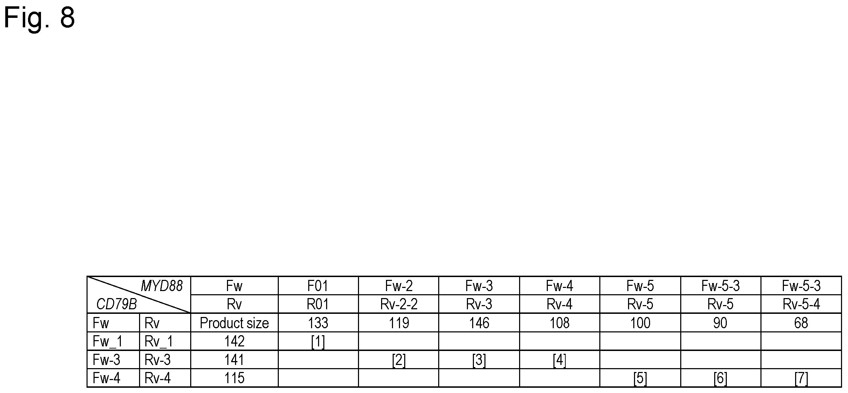

Regarding the primer set for amplifying a region comprising a gene mutation in the CD79B gene and the primer set for amplifying a region comprising a gene mutation in the MYD88 gene that are shown in Tables 7 and 8, seven types of combinations ([1] to [7]) shown in Figure 8 were tested. An optimal primer set was selected based on the amplification amount of a target nucleic acid fragment and the amplification amount of a non-specific nucleic acid amplification.

First, a reaction liquid was prepared using a wild-type model specimen or genomic DNA (gDNA) as a template. Using the prepared PCR reaction liquid, a thermal cycle of PCR was performed at 95°C for 5 minutes, followed by one cycle of 95°C for 30 seconds, a predetermined annealing temperature (for example, 59°C) for 30 seconds, and 72°C for 45 seconds. Thereafter, 72°C for 10 minutes was set, and finally 4°C was maintained. Note that an annealing temperature was 56°C, 58°C, 60°C, 62°C, or 64°C.

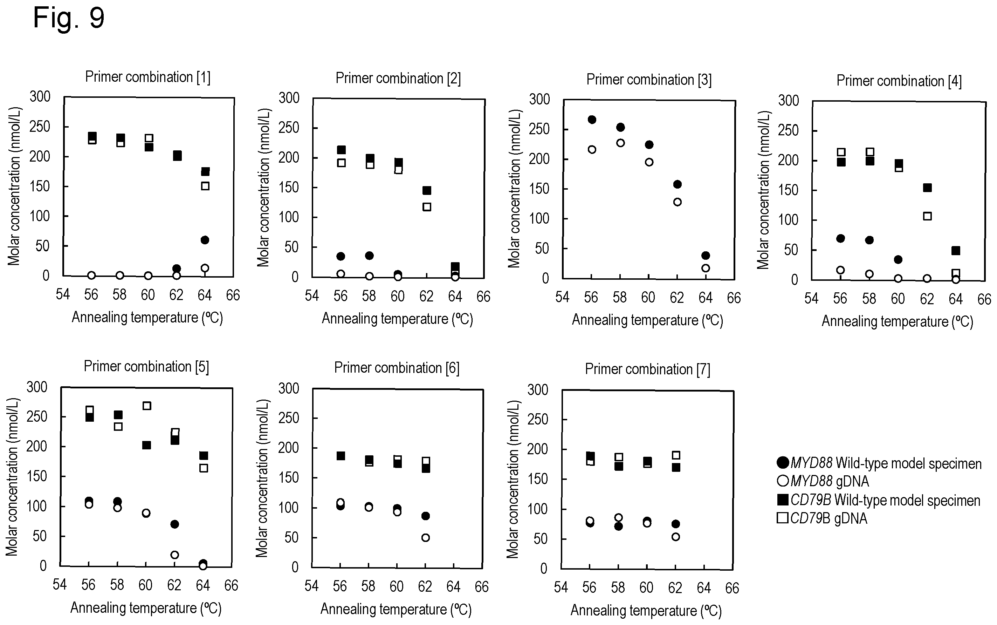

The relationship between the nucleic acid amplification amount and the annealing temperature is shown in Figure 9. As can be seen from Figure 9, the region comprising a gene mutation in the MYD88 gene and the region comprising a gene mutation in the CD79B gene can be amplified in the same manner (multiplex amplified) with both the wild-type model specimen and the genomic DNA by the combinations [5], [6], and [7].

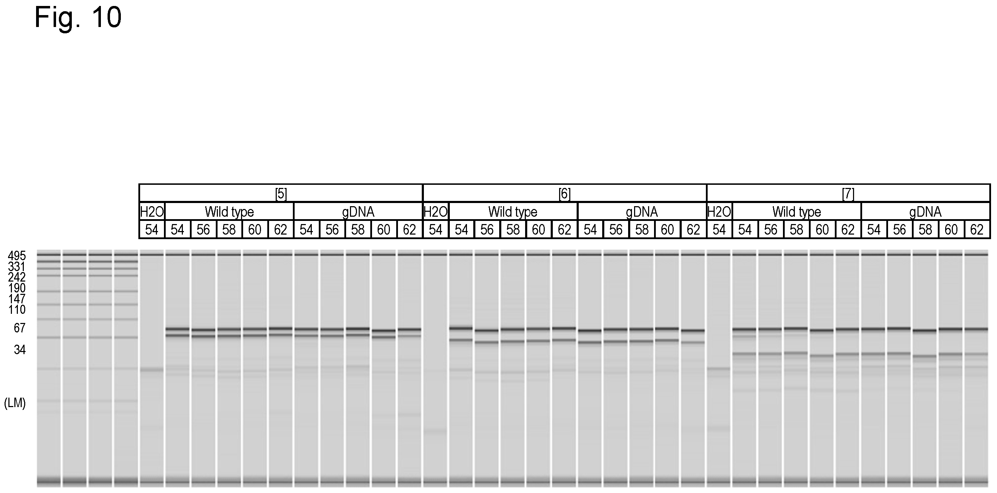

Additionally, the results of confirming a nucleic acid fragment comprised in a reaction liquid after a PCR performed with the combinations [5], [6], and [7] by electrophoresis are shown in Figure 10. As shown in Figure 10, a plurality of nucleic acid fragments caused by non-specific amplification other than target nucleic acid fragments were confirmed when the combination [7] was used. This result has revealed that the combinations [5] and [6] are better than the combination [7].

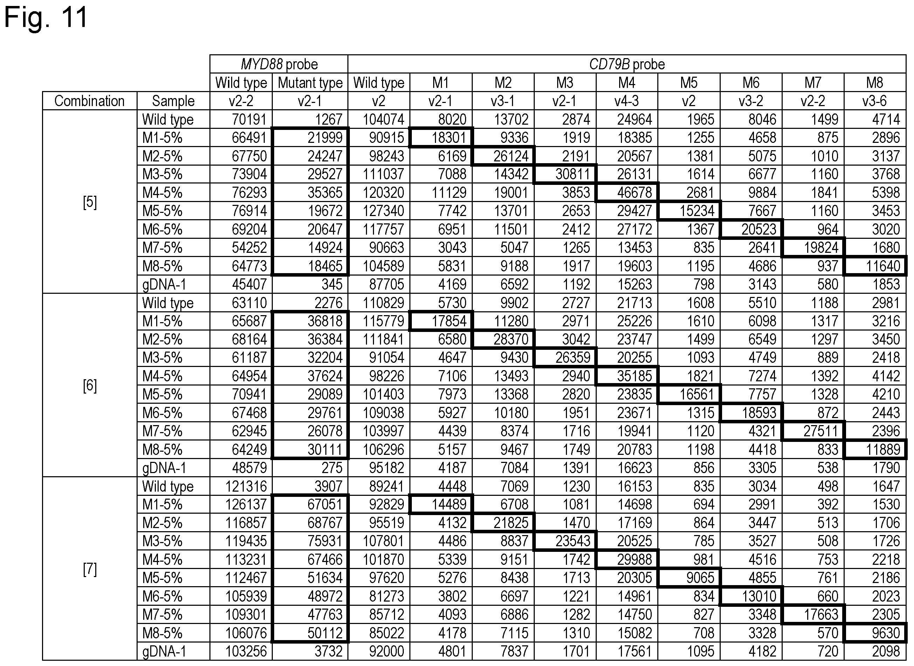

Next, a reaction liquid was prepared using a 5% mutation model specimen or genomic DNA (gDNA-1) as a template. A PCR was performed in the same manner using the reaction liquid, and fluorescence intensities based on the amplified fragments were measured. The results are shown in Figure 11. As shown in Figure 11, when the combination [5] was used, it has been revealed that a fluorescence intensity from a mutant-type probe corresponding to a mutant type in the gene mutation in the MYD88 gene is lower compared to other combinations.

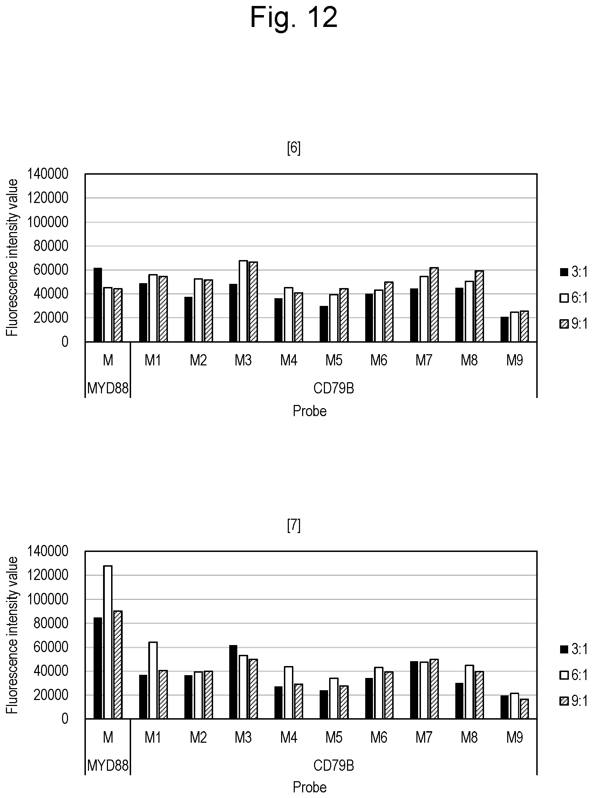

Then, regarding the combinations [6] and [7], what kind of changes occurred in fluorescence intensity values when a mixing ratio of a labeled primer and an unlabeled primer was changed was examined. The results are shown in Figure 12. As shown in Figure 12, in the case of the combination [6], the fluorescence intensity became higher as the mixing ratio of the labeled primer became higher, and a fluorescence intensity value corresponding to the ratio of the labeled primer was obtained. On the other hand, in the case of the combination [7], the ratio of the labeled primer and the trend of change in the fluorescence intensity value did not match. This was considered to be due to non-specific amplification observed when the combination [7] was used. These results have revealed that it is preferable to multiplex-amplify a region comprising a gene mutation in the CD79B gene and a region comprising a gene mutation in the MYD88 gene by the combination [6].

### <Examination of primer ratio (labeled vs unlabeled)>

In each of a primer set for amplifying a region comprising a gene mutation in the CD79B gene and a primer set for amplifying a region comprising a gene mutation in the MYD88 gene, a ratio between a labeled primer and an unlabeled primer was changed, how a fluorescence intensity value fluctuates was examined, and an optimal ratio between the labeled primer and the unlabeled primer was examined. Note that, in this Example, in the primer set for amplifying a region comprising a gene mutation in the CD79B gene, the forward primer is a labeled primer (CD79B_Fw-4), and the reverse primer is an unlabeled primer (CD79B_Rv-4). Additionally, in the primer set for amplifying a region comprising a gene mutation in the MYD88 gene, the forward primer is an unlabeled primer (MYD88_Fw-5-3), and the reverse primer is a labeled primer (MYD88_Rv-5).

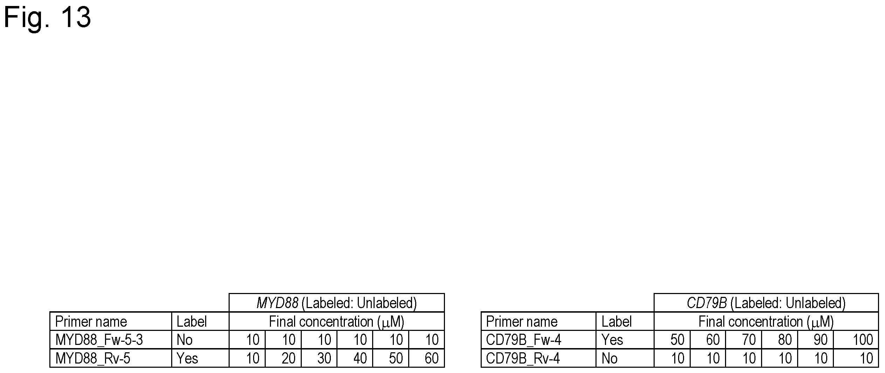

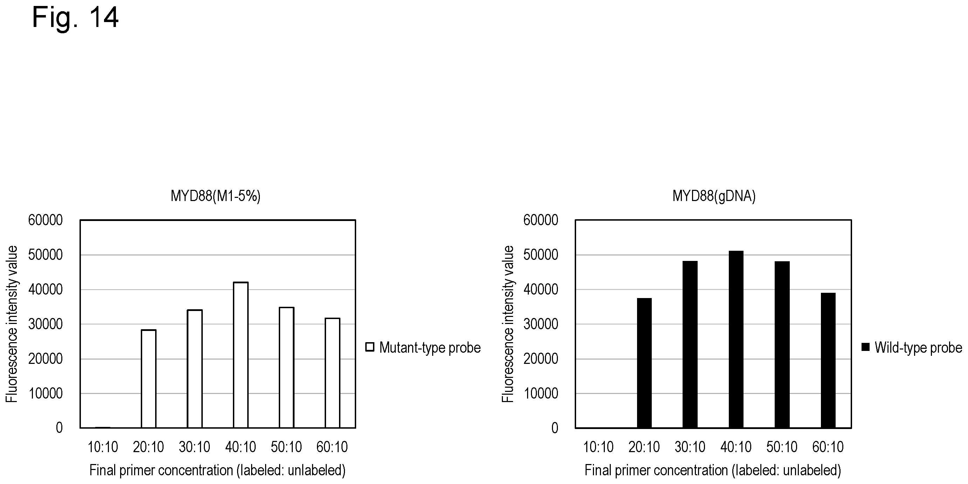

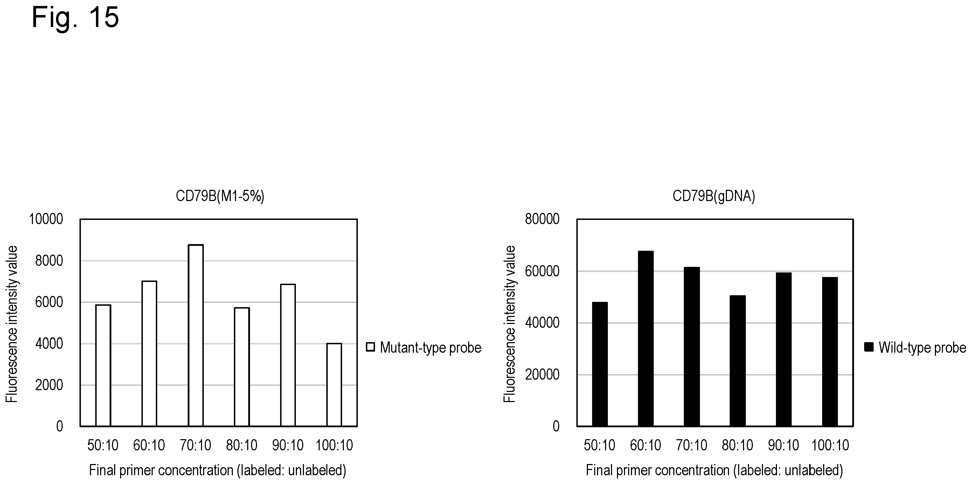

In this Example, as shown in Figure 13, the final concentrations of the labeled primers (CD79B_Fw-4 and MYD88_Rv-5) and the unlabeled primers (CD79B_Rv-4 and MYD88_Fw-5-3) comprised in a PCR reaction liquid were adjusted, and a PCR was performed in the same manner. As templates, a 5% mutation model specimen and genomic DNA were used. The results obtained when the primer set for amplifying a region comprising a gene mutation in the MYD88 gene was used are shown in Figure 14, and the results obtained when the primer set for amplifying a region comprising a gene mutation in the CD79B gene was used are shown in Figure 15.

As can be seen from Figure 14, excellent fluorescence intensity values were achieved in the range where the final concentration of the labeled primer for amplifying a region comprising a gene mutation in the MYD88 gene was 2 times (that is, labeled primer: unlabeled primer = 20:10) to 6 times as high as the final concentration of the unlabeled primer. In particular, more excellent fluorescence intensity values were achieved in the range of from 3 times to 5 times, and a most excellent fluorescence intensity value was achieved at 4 times.

Additionally, as can be seen from Figure 15, excellent fluorescence intensity values were achieved in the range where the final concentration of the labeled primer for amplifying a region comprising a gene mutation in the CD79B gene was 5 times to 10 times as high as the final concentration of the unlabeled primer. In particular, more excellent fluorescence intensity values were achieved in the range of from 6 times to 7 times, and a most excellent fluorescence intensity value was achieved at 7 times.

### <Examination of optimal blocking nucleic acid>

From the blocking nucleic acids related to a gene mutation in the MYD88 gene shown in Table 9, and the blocking nucleic acids related to a gene mutation in the CD79B gene shown in Table 10, an optimal blocking nucleic acid was selected based on a function as a blocking nucleic acid. A blocking nucleic acid specifically hybridizes to a wild-type nucleic acid fragment among nucleic acid fragments obtained by amplifying a region comprising a gene mutation, and has the function of preventing a wild-type nucleic acid fragment from non-specifically hybridizing to a mutant-type probe. Accordingly, in selecting an optimal blocking nucleic acid, non-specific hybridization between a wild-type nucleic acid fragment and a mutant-type probe in the presence of the blocking nucleic acid can be determined based on a fluorescence intensity from the mutant-type probe. Additionally, whether a blocking nucleic acid does not inhibit specific hybridization between a mutant-type nucleic acid fragment and a mutant-type probe in the presence of the blocking nucleic acid can be determined based on a fluorescence intensity from the mutant-type probe.

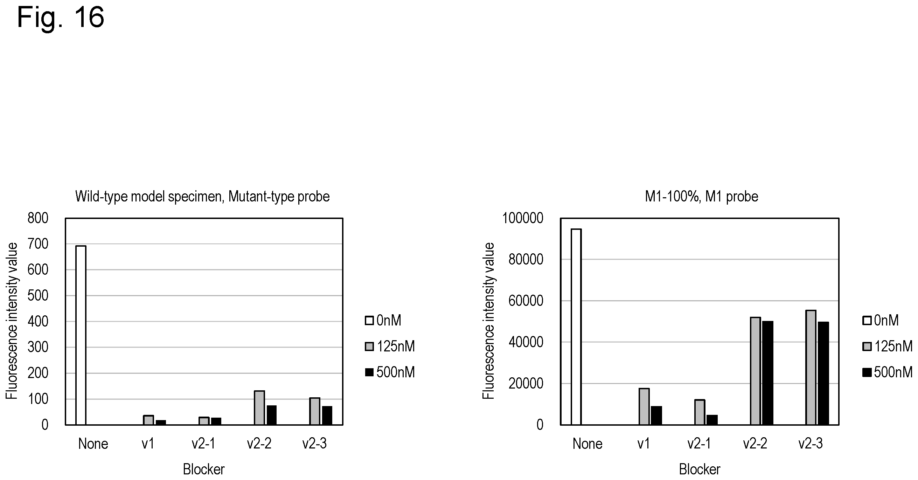

Regarding the blocking nucleic acid related to a gene mutation in the MYD88 gene shown in Table 9, a wild-type model specimen and the mutant-type probe (MYD88_M_v2-1) were hybridized in the presence of the blocking nucleic acid (125 nM or 500 nM), and a fluorescence intensity from the mutant-type probe was measured. Additionally, a 100% mutation model specimen and the mutant-type probe (MYD88_M_v2-1) were hybridized in the presence of the blocking nucleic acid, and a fluorescence intensity from the mutant-type probe was measured. The results are shown in Figure 16.

As can be seen from Figure 16, non-specific hybridization between the wild-type model specimen and the mutant-type probe was successfully suppressed by all the tested blocking nucleic acids, but it has been revealed that v1 and v2-1 among the blocking nucleic acids inhibited specific hybridization between the 100% mutation model specimen and the mutant-type probe. Then, which of v2-2 and v2-3 functions as a more excellent blocking nucleic acid was verified using a 5% mutation model specimen.

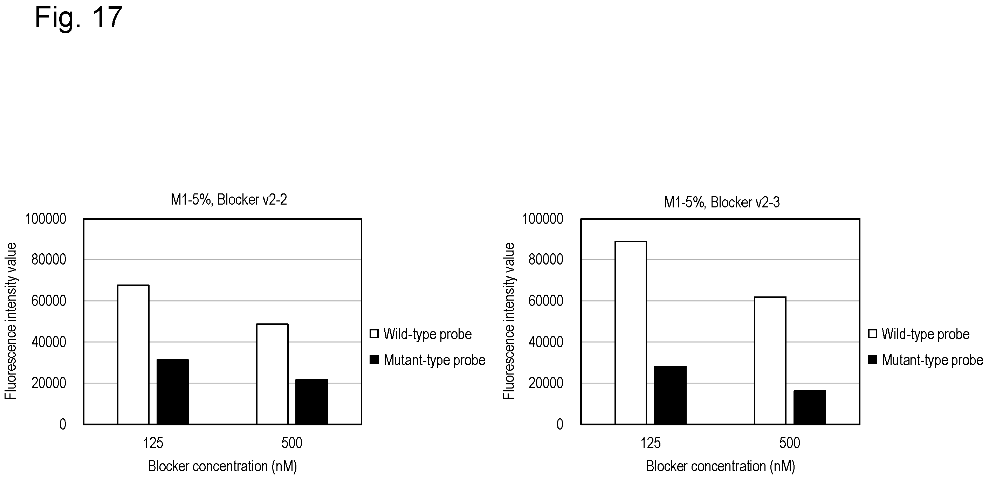

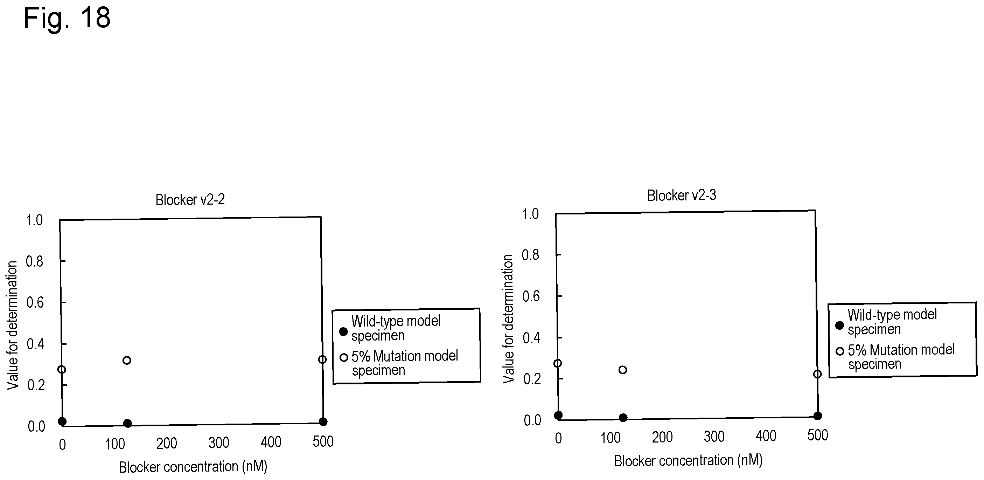

That is, in the presence of v2-2 or v2-3 (125 nM or 500 nM), the 5% mutation model specimen were hybridized with the wild-type probe (MYD88_W_v2-1) and with the mutant-type probe (MYD88_M_v2-1). Fluorescence intensities from the wild-type probe and the mutant-type probe were measured. Furthermore, values for determination were calculated according to the above-described Formula from the measured fluorescence intensity values. The results of measuring fluorescence intensities are shown in Figure 17, and the results of calculating values for determination are shown in Figure 18.

As can be seen from Figures 17 and 18, the fluorescence intensity from the wild-type probe was lower when v2-2 was used than when v2-3 was used. It has been revealed that v2-2 has an excellent function as a blocking nucleic acid compared to v2-3. Based on this result, v2-2 among the blocking nucleic acids shown in Table 9 was selected as an optimal blocking nucleic acid related to a gene mutation in the MYD88 gene. Additionally, these results have revealed that, even when a blocking nucleic acid related to a gene mutation in the MYD88 gene was not used (that is, in the case of 0 nM), it has been revealed that a value for determination obtained with use of the wild-type model specimen and a value for determination obtained with use of the 5% mutation model specimen were sufficiently separate. The above results have revealed that the concentration of a blocking nucleic acid related to a gene mutation in the MYD88 gene is preferably set to 0 to 500 nM.

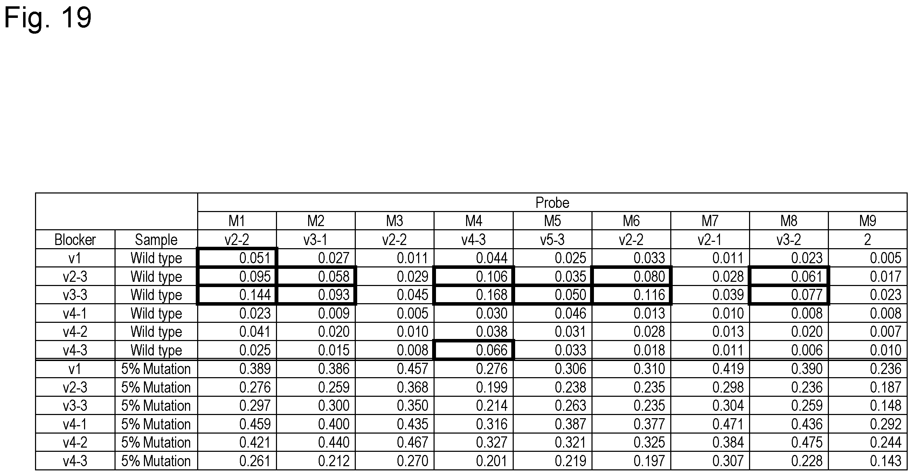

Next, regarding a blocking nucleic acid related to a gene mutation in the CD79B gene shown in Table 10, a wild-type model specimen or a 5% mutation model specimen was hybridized with the wild-type probe (CD79B_W_v3-1) and with the mutant-type probe (CD79B_M1_v2-2, CD79B_M2_v3-1, CD79B_M3-v2-2, CD79B_M4_v4-3, CD79B_M5_v5-3, CD79B_M6_v2-2, CD79B_M7_v2-1, CD79B_M8_v3-2, and CD79B_M9-2) in the presence of a blocking nucleic acid, fluorescence intensities from the wild-type probe and the mutant-type probe were measured, and values for determination were calculated according to the above-described Formula. The results are shown in Figure 19.

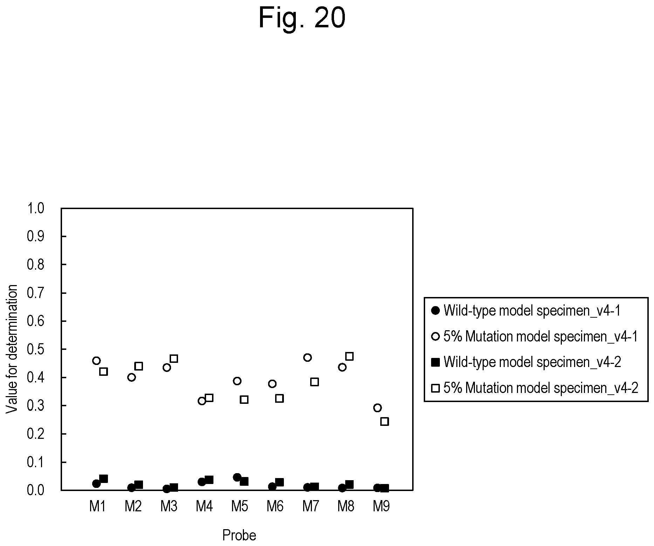

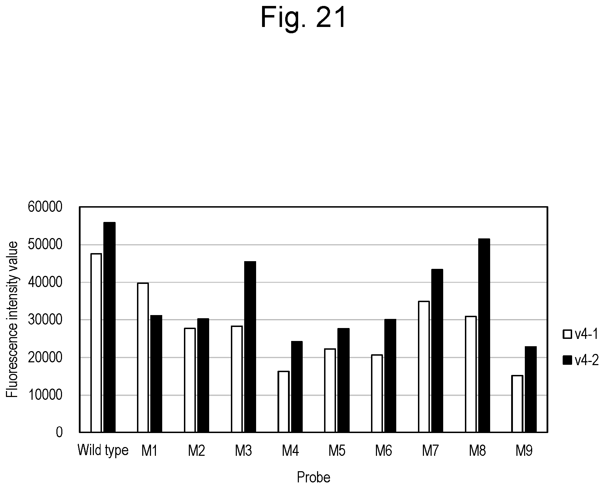

As shown in Figure 19, when the wild-type model specimen was used, the cases where the values for determination in all the mutant-type probes were 0.05 or less were only cases where the blocking nucleic acids v4-1 and v4-2 were used. Regarding the blocking nucleic acids v4-1 and v4-2, values for determination and fluorescence intensity values in each mutant-type probe calculated using the wild-type model specimen or the 5% mutation model specimen are shown in Figures 20 and 21, respectively. As can be seen from Figures 20 and 21, comparison between values for determination for v4-1 and v4-2 has revealed that both were almost equal, but comparison between fluorescence intensity values for v4-1 and v4-2 has revealed that v4-2 shows a higher value. Based on this result, v4-2 among the blocking nucleic acids shown in Table 10 was selected as an optimal blocking nucleic acid related to a gene mutation in the CD79B gene.

### <Examination of blocking nucleic acid concentration>

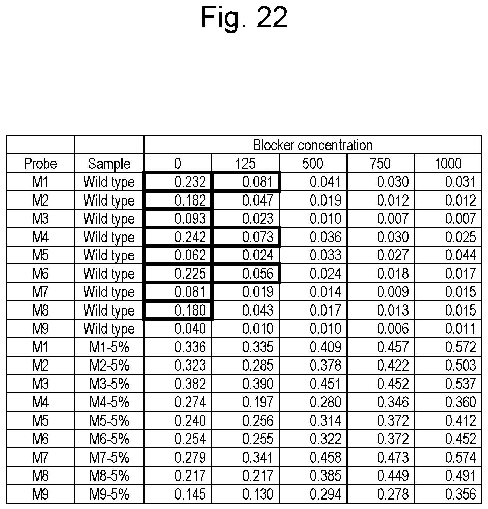

Regarding v4-2 selected as a blocking nucleic acid related to a gene mutation in the CD79B gene, a wild-type model specimen or a 5% mutation model specimen was hybridized with the wild-type probe (CD79B_W_v3-1) and with the mutant-type probe (CD79B_M1_v2-2, CD79B_M2_v3-1, CD79B_M3-v2-2, CD79B_M4_v4-3, CD79B_M5_v5-3, CD79B_M6_v2-2, CD79B_M7_v2-1, CD79B_M8_v3-2, and CD79B_M9-2) in the presence of the blocking nucleic acid at different concentrations, fluorescence intensities from the wild-type probe and the mutant-type probe were measured, and values for determination were calculated according to the above-described Formula. The results are shown in Figure 22.

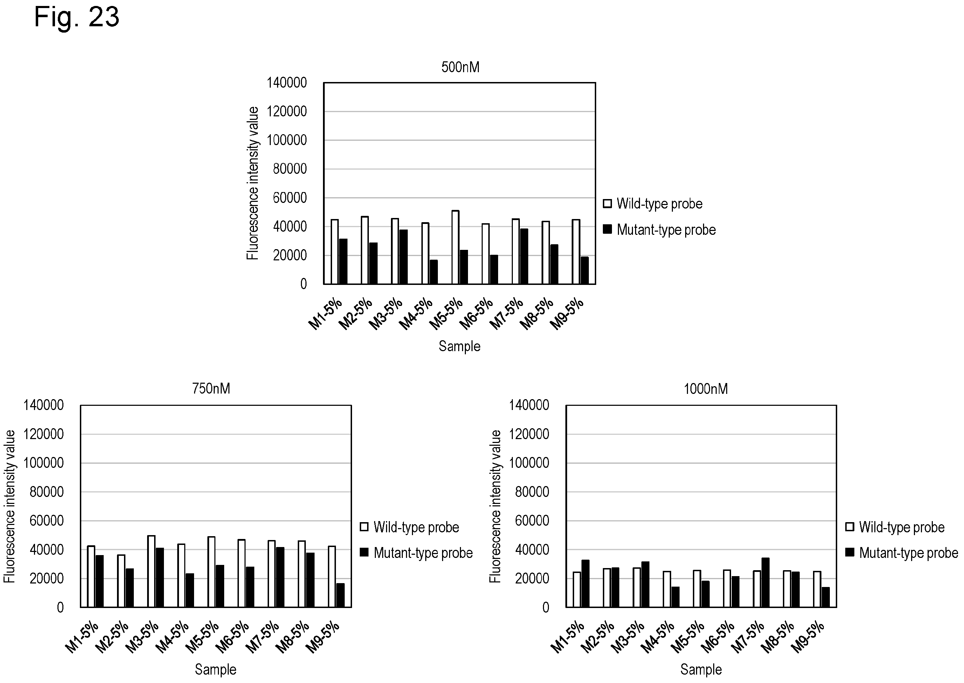

As shown in Figure 22, when the concentration of a blocking nucleic acid was 125 nM, a plurality of mutant-type probes had values for determination exceeding 0.05 when the wild-type model specimen was used. This result has revealed that the concentration of the blocking nucleic acid is preferably higher than 125 nM. Additionally, Figure 23 shows the result of measuring fluorescence intensity from each mutant-type probe when the concentration of the blocking nucleic acid was 500 nM, 750 nM, or 1000 nM, and when the 5% mutation model specimen was used. This result has revealed that when the concentration of the blocking nucleic acid is 1000 nM, the fluorescence intensity value is lower than when the concentration is 500 nM or 750 nM. These results have suggested that the concentration of the blocking nucleic acid is preferably 500 nM or 750 nM.

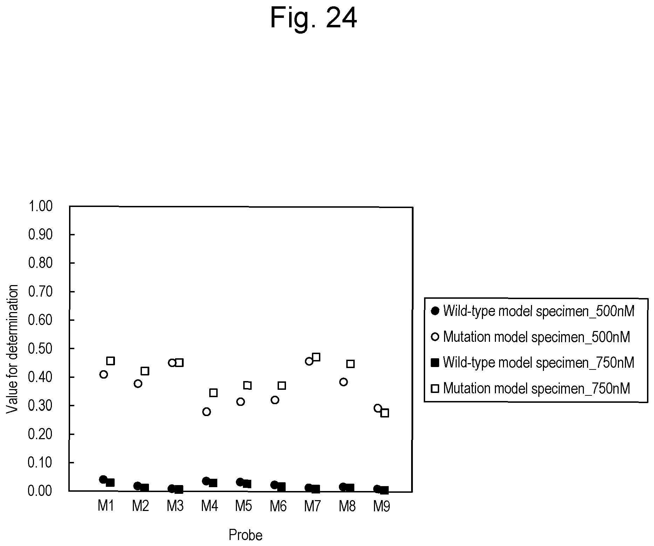

In order to confirm the above results, the value for determination of each mutant-type probe when a wild-type model specimen or a 5% mutation model specimen was used, and when the concentration of the blocking nucleic acid was 500 nM or 750 nM was calculated. The results are shown in Figure 24. As can be seen from Figure 24, in either case where the concentration of the blocking nucleic acid was 500 nM or 750 nM, the value for determination when the wild-type model specimen was used and the value for determination when the 5% mutation model specimen was used were sufficiently separated, and it has been revealed that the wild type and each mutant type can be accurately detected. In particular, when the concentration of the blocking nucleic acid was 750 nM, the value for determination when the wild-type model specimen was used and the value for determination when the 5% mutation model specimen was used were separated more largely compared to when the concentration was 500 nM.

The above results have revealed that the concentration of the blocking nucleic acid can be in the range of from 125 nM to 1000 nM, and is preferably from 500 to 750 nM. As an example, it has been revealed that the concentration of the blocking nucleic acid is most preferably 750 nM.

All publications, patents, and patent applications cited herein are incorporated herein by reference in their entirety.

## Claims

1. A gene mutation evaluation kit for use in diagnosing a disease associated with a gene mutation in the MYD88 gene and/or a gene mutation in the CD79B gene, comprising:
a primer set for a MYD88 gene mutation for amplifying a nucleic acid region comprising a gene mutation in the MYD88 gene; and a primer set for a CD79B gene mutation for amplifying a nucleic acid region comprising a gene mutation in the CD79B gene; and
a MYD88 gene mutant-type probe and a MYD88 gene wild-type probe related to the gene mutation in the MYD88 gene, and a CD79B gene mutant-type probe and a CD79B gene wild-type probe related to the gene mutation in the CD79B gene.

2. The gene mutation evaluation kit of claim 1, wherein
the gene mutation in the MYD88 gene is a substitution mutation for leucine at position 265 with proline, and
the gene mutation in the CD79B gene is a missense mutation and/or nonsense mutation for the tyrosine residue at position 196 and a silent mutation for the tyrosine residue.

3. The gene mutation evaluation kit of claim 1, wherein
the primer set for a MYD88 gene mutation is a set of a forward primer for the MYD88 gene consisting of the base sequence of SEQ ID NO: 79 and a reverse primer for the MYD88 gene consisting of the base sequence of SEQ ID NO: 78, and
the primer set for a CD79B gene mutation is a set of a forward primer for the CD79B gene consisting of the base sequence of SEQ ID NO: 85 and a reverse primer for the CD79B gene consisting of the base sequence of SEQ ID NO: 86.

4. The gene mutation evaluation kit of claim 3, wherein
the reverse primer for the MYD88 gene has a label and has a concentration 2 to 6 times higher than that of the forward primer for the MYD88 gene, and
the forward primer for the CD79B gene has a label and has a concentration 5 to 10 times higher than that of the reverse primer for the CD79B gene.

5. The gene mutation evaluation kit of claim 1, wherein the MYD88 gene mutant-type probe, the MYD88 gene wild-type probe, the CD79B gene mutant-type probe and the CD79B gene wild-type probe comprise the base sequences shown in Table 1.
**[Table 1]**
| Probe type | Probe base sequence | SEQ ID NO |
|---|---|---|
| MYD88 gene wild-type probe | AGCGACTGATCCCCAT | 2 |
| MYD88 gene mutant-type probe | GAAGCGACCGATCCC | 6 |
| CD79B gene wild-type probe | CTTACCTCGTAGGTGTGAT | 10 |
| CD79B gene mutant-type probe M1 | CTTACCTCGTGGGTGTGA | 14 |
| CD79B gene mutant-type probe M2 | CTTACCTCGTTGGTGTGA | 19 |
| CD79B gene mutant-type probe M3 | CTTACCTCGTCGGTGTGA | 24 |
| CD79B gene mutant-type probe M4 | TTACCTCGGAGGTGTGAT | 32 |
| CD79B gene mutant-type probe M5 | CTCGCAGGTGTGATCTT | 38 |
| CD79B gene mutant-type probe M6 | CTTACCTCGAAGGTGTGAT | 41 |
| CD79B gene mutant-type probe M7 | CTTACCTCCTAGGTGTGATCT | 50 |
| CD79B gene mutant-type probe M8 | TCTCCTTACCTCTTAGGTGTG | 57 |
| CD79B gene mutant-type probe M9 | TCTCCTTACCTCATAGGTGTG | 66 |

6. The gene mutation evaluation kit of claim 1, further comprising a blocking nucleic acid for MYD88 that hybridizes to a wild-type nucleic acid fragment among the nucleic acid fragments amplified with the primer set for MYD88 gene mutation, and/or a blocking nucleic acid for CD79B that hybridizes to a wild-type nucleic acid fragment among the nucleic acid fragments amplified with the primer set for CD79B gene mutation.

7. The gene mutation evaluation kit of claim 6, wherein
the blocking nucleic acid for MYD88 consists of the base sequence of SEQ ID NO: 89, and
the blocking nucleic acid for CD79B consists of the base sequence of SEQ ID NO: 95.

8. The gene mutation evaluation kit of claim 6, wherein
the blocking nucleic acid for MYD88 is prepared in a hybridization solution at a concentration in the range of 0 to 500 nM, and
the blocking nucleic acid for CD79B is prepared in a hybridization solution at a concentration in the range of 125 to 1000 nM.
